(19) Europäisches Patentamt
European Patent Office
Office européen des brevets

(11) Publication number: 0 197 409 B1

(12) # EUROPEAN PATENT SPECIFICATION

(45) Date of publication of the patent specification: 12.09.90

(51) Int. Cl.[5]: C07D 501/36, C07D 501/46, C07D 501/34, C07D 277/20, A61K 31/545

(21) Application number: 86103996.4

(22) Date of filing: 24.03.86

(54) **Cephalosporin derivatives.**

(30) Priority: 01.04.85 JP 68866/85
17.05.85 JP 105704/85
04.07.85 JP 147359/85
27.07.85 JP 166259/85
10.03.86 US 838309

(43) Date of publication of application: 15.10.86 Bulletin 86/42

(45) Publication of the grant of the patent: 12.09.90 Bulletin 90/37

(84) Designated Contracting States: AT BE CH DE FR GB IT LI LU NL SE

(56) References cited:
EP-A- 0 075 805
EP-A- 0 150 507
FR-A- 2 456 109

CHEMICAL ABSTRACTS, vol. 102, no. 13, 1st April 1985, page 681, abstract no. 113169e, Columbus, Ohio, US; & JP-A-59 167 576

(73) Proprietor: MOCHIDA PHARMACEUTICAL CO., LTD., 7, Yotsuya 1-chome, Shinjuku-ku Tokyo 160(JP)

(72) Inventor: Ohnishi, Haruo, 6-4-14, Higashifunabashi, Funabashi-shi Chiba-ken(JP)
Inventor: Kosuzume, Hiroshi, 3-10-6, Fuyoudai, Mishima-shi Shizuoka-ken(JP)
Inventor: Mizota, Masahiro, 46-7, Fujimidai, Mishima-shi Shizuoka-ken(JP)
Inventor: Suzuki, Yasuo, 234-29, Oaza Sashima, Kawaguchi-shi Saitama-ken(JP)
Inventor: Mochida, Ei, 2-5-4, Komagome, Toshima-ku Tokyo(JP)

(74) Representative: Eitle, Werner, Dipl.-Ing. et al, Hoffmann, Eitle & Partner Patentanwälte Arabellastrasse 4, D-8000 München 81(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The present invention relates to novel cephalosporin derivatives, processes for producing cephalosporin derivatives, and compositions containing cephalosporin derivatives for treating and/or preventing infectious diseases.

Developments of cephalosporin derivatives have been remarkable. Some cephalosporin derivatives have been developed which have excellent antibacterial activity against Gram-negative bacteria. However, the antibacterial activity of these cephalosporin derivatives against Gram-positive bacteria is rather poor. Several cephalosporin antibiotics have been used for the treatment of Gram-positive bacteria infections and the increase of Gram-positive bacteria resistant to cephalosporin antibiotics, for example, methicillin-resistant Staphylococcus aureus (MRSA), has become widely known year by year.

JP-A 59 167 576 describes various cephem derivatives in which the alkoxyimino residue is substituted by a 3,4-dihydroxyphenyl group. Such compounds are noted as having antibacterial activity.

From the foregoing background, it has been desired to develop cephalosporin derivatives having a strong antibacterial activity against Gram-positive bacteria while retaining a sufficient antibacterial activity against Gram-negative bacteria.

## SUMMARY OF THE INVENTION

An object of the present invention is to provide novel cephalosporin derivatives and salts, hydrates and salts of hydrates thereof.

Another object of the present invention is to provide processes for producing novel cephalosporin derivatives.

A further object of the present invention is to provide compositions for preventing and/or treating infectious diseases which comprise novel cephalosporin derivatives as active components.

A further object of the present invention is to provide intermediate compounds in the synthesis of cephalosporin derivatives and processes for producing such intermediate compounds.

The present invention is based on the selection of groups containing a condensed heterocyclic ring, particularly a triazolopyrimidine ring as a substituent at the 3-position of the cephem skeleton, and of groups containing a catechol moiety, particularly a catechol carboxymethyloxyimino moiety or a catechol carboxymimino moiety, as substituents at the 7-position of the cephem skeleton.

The compounds of the present invention containing these substituents have a wide antibacterial spectrum against Gram-negative bacteria and Gram-positive bacteria including methicillin-resistant Staphylococcus aureus. These compounds are extremely useful for the treatment of infectious diseases.

## DETAILED DESCRIPTION OF THE INVENTION

As a result of extensive investigations concerning development of cephalosporin derivatives having a satisfactory antibacterial activity against Gram-negative bacteria and also having strong antibacterial activity against Gram positive bacteria, the present inventors have found that cephalosporin derivatives represented by the general formula (I) satisfy these requirements and, have accomplished the present invention.

The present invention is based on the selection of groups containing a condensed heterocyclic ring, particularly a triazolopyrimidine ring as a substituent at the 3-position of the cephem skeleton, and of groups containing a catechol moiety, particularly a catechol carboxymethyloxyimino moiety or a catechol carboxyimino moiety, as substituents at the 7-position of the cephem skeleton.

The present invention is directed to cephalosporin derivatives represented by the general formula (I):

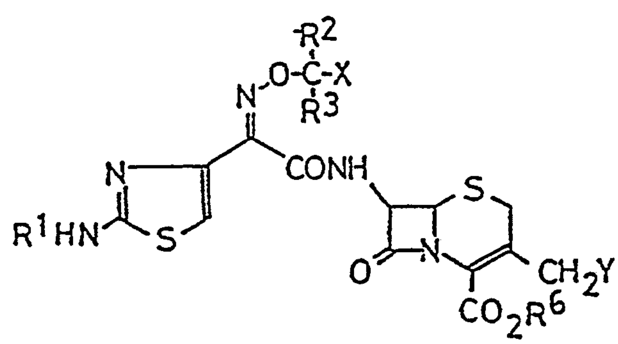

(I)

or a pharmaceutically or veterinarily acceptable non-toxic salt, or solvate, or a non-toxic salt of a solvate thereof; wherein $R^1$ represents a hydrogen atom or a conventional amino-protecting group, $R^2$ represents a hydrogen atom, a methyl group, a carboxyl group, or a conventionally protected carboxyl

group, $R^3$ represents a hydrogen atom or a methyl group, $R^6$ represents a hydrogen atom or a conventional carboxyl protecting group, X represents a group:

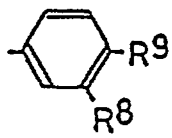

wherein $R^8$ and $R^9$ are the same or different and represent hydroxy groups or acetoxy groups, and Y represents a group:

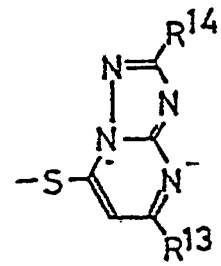

wherein $R^{13}$ represents a methyl group, and $R^{14}$ represents a carboxyl group or a conventionally protected carboxyl group.

The present invention is also directed to a process for preparing above-mentioned cephalosporin derivatives. The present invention is further directed to pharmaceutical compositions for treating and/or preventing infectious diseases characterized by containing these cephalosporin derivatives as active components.

In the cephalosporin derivatives of the present invention represented by the general formula (I), it is known that the aminothiazole moiety as the substituent at the 7-position thereof exhibits tautomerism as shown below:

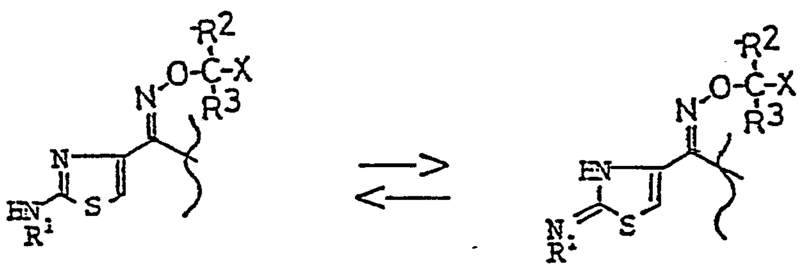

wherein $R^1$, $R^2$, $R^3$, and X have the same significance as defined above. In the present invention, the aminothiazole moiety is expressed as including both isomers since both are generally deemed to be the same substance. Accordingly, the compounds of the present invention represented by the general formula (I) also include both of these tautomeric isomers.

The compounds represented by the general formula (I) may form acid or base addition salts. Typical examples of base addition salts of the compounds represented by the general formula (I) include pharmacologically acceptable salts such as alkali metal salts such as sodium salts, potassium salts, etc.; alkaline earth metal salts such as calcium salts, etc.; salts of organic bases such as ammonium salts, benzylamine salts, diethylamine salts, etc.; salts of amino acids such as arginine salts, lysine salts, etc. These salts of the compounds may be a mono-salts, di-salts or tri-salts. In the case of mono-salts or di-salts, the salts may be salts of the carboxyl group at the 2-position and/or salts of the carboxyl or sulfoxy group contained in the substituents at the 3-position, and/or salts of the carboxyl group in the acyl group at the 7-position, of the cephem skeleton.

Typical examples of acid addition salts of the compounds represented by the general formula (I) include pharmacologically acceptable salts, such as salts of inorganic acids such as hydrochlorides, hydrobromides, sulfates, phosphates, etc.; salts of organic acids such as acetates, citrates, maleates, tartarates, benzoates, ascorbates, ethanesulfonates, toluenesulfonates, etc.; salts of amino acids such as aspartates, glutamates, etc. The compounds of the present invention represented by the general formula (I) are present as the syn-isomer.

In the compounds of the present invention represented by the general formula (I), the amino-protecting groups may be selected from acyl groups such as formyl, acetyl, chloroacetyl, t-butoxycarbonyl,

benzyloxycarbonyl, etc.; or aralkyl groups such as benzyl, diphenylmethyl, triphenylmethyl, etc. Trimethylsilyl group may also be used as an amino-protecting group. The carboxyl-protecting groups may be selected from alkyl esters such as methyl ester, ethyl ester, t-butyl ester, etc.; or aralkyl esters such as benzyl ester, diphenylmethyl ester, triphenylmethyl ester, etc.; or trimethylsilyl ester. Inorganic or organic bases may also be used as carboxyl-protecting groups. Collectively taking account of various operations, synthesis of thus protected products, and conditions for the removal of protecting groups, it is preferred to use a triphenylmethyl group as the amino-protecting group and a diphenylmethyl group as the carboxyl-protecting group.

The compounds of the present invention represented by the general formula (I) can be produced as follows. Namely;

Process A

The compounds of the present invention represented by the general formula (I) can be produced by reacting compounds represented by the general formula (II):

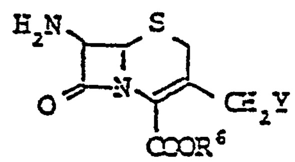

(II)

wherein $R^6$ and Y have the same significance as defined above, with compounds represented by the general formula (III):

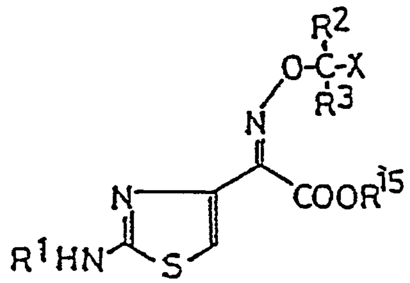

(III)

wherein $R^1$, $R^2$, $R^3$, and X have the same significance as defined above, and $R^{15}$ represents a hydrogen atom or a carboxyl-protecting group.

The compounds represented by the general formula (II) may be reacted with the compounds represented by the general formula (III) using suitable condensing agents, for example, N,N-dicyclohexylcarbodiimide, N-ethyl-5-phenylisoxazolium-3'-sulfonate, etc. Alternatively, the compounds represented by the general formula (III) may be converted into appropriate reactive derivatives prior to the reaction with the compounds represented by the general formula (II). The appropriate reactive derivatives may be, for example, acid halides (e.g., acid chlorides), azides, acid anhydrides, particularly mixed acid anhydride with strong acids, active esters (e.g., N-hydroxysuccinimide ester) or active amides (e.g., imidazolide, triazolide).

The reaction between the compounds represented by the general formula (II) and the compounds represented by the general formula (III) may be carried out generally in an inert organic solvent such as dioxane, tetrahydrofuran, acetonitrile, chloroform, methylene chloride, ethyl acetate, dimethylformamide, etc., if necessary and desired, in the presence of deacidifying agents. The reaction may also be carried out in an aqueous solution, preferably in the presence of deacidifying agents. As the deacidifying agents, triethylamine, diethylaniline, and the like may be used in the organic solvent system, and aqueous alkalis, preferably sodium hydroxide, sodium hydrogen carbonate (sodium bicarbonate), potassium carbonate, and the like may be used in the aqueous system.

The reaction may be carried out at temperatures ranging from about –30°C to 30°C, and preferably from –10°C to 10°C.

If necessary and desired, the protecting groups may be removed from thus obtained cephalosporin derivatives represented by the general formula (I).

The compounds represented by the general formula (II) used in the process of the present invention can be prepared by reacting known 7-amino-cephalosporanic acid with heterocyclic thiols or pyridine derivatives corresponding to group Y, for example, 2-carboxy-7-mercapto-5-methyl-s-triazolo[1,5-a]pyrimidine (Japanese Patent Application 247 251/1983) or carboxyl-protected derivatives thereof, in a solvent such as alcohols, dimethylformamide, acetonitrile, or water etc. In the case that the reaction is carried out in organic solvents, it is preferred that the reaction be performed in the presence of Lewis acids such as boron trifluoride-ether complexes, etc. Further in the case that water is used as the solvent, the reaction can be carried out in the presence of an appropriate amount of aqueous alkalis such as sodium hydrogen carbonate, potassium carbonate, etc., or using buffers having a pH of 6.0 to 7.8 as the solvent.

The reaction temperature may be in the range of about 40°C to about 80°C, and preferably from 55°C to 65°C.

If necessary and desired, the protecting groups may be removed from thus obtained compounds represented by the general formula (II).

Process B

The compounds represented by the general formula (I) can be produced by reacting compounds represented by the general formula (IV):

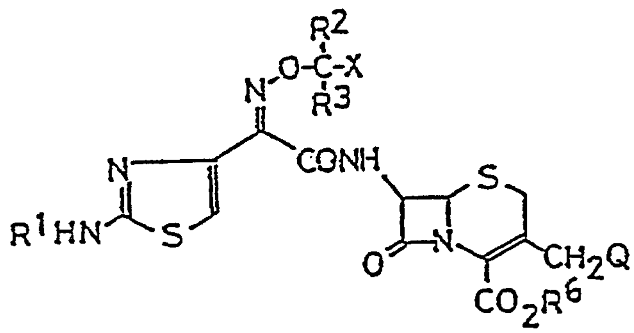

(IV)

wherein $R^1$, $R^2$, $R^3$, and X have the same significance as defined above, and Q represents a chlorine atom, a bromine atom, an iodine atom, or an acetoxy group, with compounds represented by the general formula (V):

$$H\text{–}Y \qquad (V)$$

wherein Y has the same significance as defined above.

The reaction between the compounds represented by the general formula (IV) and the compounds represented by the general formula (V) may be carried out in an organic solvent such as alcohols, dimethylformamide, dimethylsulfoxide, dichloromethane, etc. or mixture thereof, or in an aqueous system. Preferably, the reaction of the compounds represented by the general formula (IV) wherein Q represents an acetoxy group, and the compounds represented by the general formula (V) may be carried out in the presence of an appropriate amount of aqueous alkalis, e.g., sodium hydrogen carbonate or potassium carbonate, or carried out in a buffer solution at a pH in the range of 6.0 to 7.8, at temperatures in the range of about 40°C to about 80°C, and preferably at from 55 to 65°C. Preferably, the reaction of the compounds represented by the general formula (IV) wherein Q represents a halogen atom, and the compounds represented by the general formula (V) may be carried out in an organic solvent at temperatures in the range of about –30°C to 30°C, and preferably at from –10°C to 10°C.

If necessary and desired, the protecting groups may be removed from thus obtained cephalosporin derivatives represented by the general formula (I).

The compounds represented by the general formula (IV) wherein Q represents an acetoxy group or a chlorine atom, used in the process of the present invention can be prepared by reacting the compounds represented by the general formula (III) with known 7-amino-cephalosporanic acid or carboxyl-protected derivatives thereof, or with known 3-chloromethyl-7-amino-cephalosporanic acid or carboxyl-protected derivatives thereof by the same manner as described in Process A. The compounds represented by the general formula (IV) wherein Q represents a bromine atom or an iodine atom can be prepared by reacting the compounds represented by the general formula (IV) wherein Q represents a chlorine atom with alkali bromides or iodides such as potassium bromide, potassium iodide, sodium bromide or sodium iodide in an inert organic solvent such as acetone or methylethylketone. If necessary and desired, the reaction may be carried out in the darkness. The reaction temperature may be in the range of about –10°C to 50°C and preferably 0°C to 20°C. If necessary and desired, the protecting groups may be removed from thus obtained cephalosporin derivatives represented by the general formula (IV).

Process C

The compounds represented by the general formula (I) can be produced by reacting compounds represented by the general formula (VI)

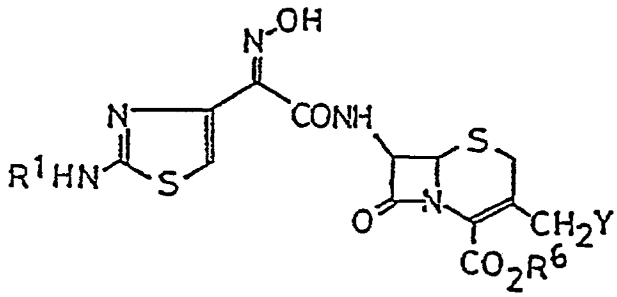

(VI)

wherein $R^1$, $R^6$ and Y have the same significance as defined above, with compounds represented by the general formula (VII)

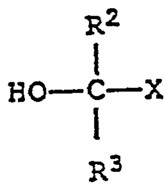

(VII)

wherein $R^2$, $R^3$ and X have the same significance as defined above. The reaction can be carried out by reacting the compounds represented by the general formula (VII) with the compounds represented by the general formula (VI) using a suitable condensing agent such as N,N-dicyclohexylcarbodiimide, triphenylphosphine, diethyl azodicarboxylate, or by reacting the compounds represented by the general formula (VI) with appropriate reactive derivatives of the compounds represented by the general formula (VII); when the compounds represented by the general formula (VII) are acids, they may be converted into acid halides, acid anhydrides or mixed acid anhydrides which are preferably prepared with strong acids, and when the compounds represented by the general formula (VII) are alcohols, they may be converted into alkyl halides or aralkyl tosylates. In view of reactivity, operability, etc., particularly preferred is the process in which the compounds represented by the general formula (VII) are converted into acid halides or aralkyl halides, and reacted with the compounds represented by the general formula (VI).

The reaction between the compounds represented by the general formula (VI) and the compounds represented by the general formula (VII) may be carried out generally in an inert organic solvent such as dioxane, tetrahydrofuran, acetonitrile, chloroform, methylene chloride, acetone, ethyl acetate, or dimethylformamide or in water or in water containing organic solvents, preferably in the presence of deacidifying agents. As the deacidifying agents, triethylamine, diethylamine, etc. may be used in an organic solvent system, and aqueous alkalis, preferably, sodium hydrogen carbonate, sodium carbonate, potassium carbonate, etc. may be used in an aqueous system.

The reaction may be carried out at temperatures ranging from about –30°C to 30°C, and preferably at from –10°C to 10°C.

If necessary and desired, the protecting groups may be removed from thus obtained cephalosporin derivatives represented by the general formula (I).

The compounds represented by the general formula (VII) may be prepared from corresponding precursor by oxidation. For example, 3,4-diacetoxy-2-methyl benzoic acid can be prepared from 3,4-diacetoxy-2-methyl acetophenone by haloform reaction; 3,4-carbonyldioxymandelic acid or 3,4-dihydroxymandelic acid can be prepared from piperonal by conversion into α-chloro-3,4-carbonyldioxyphenylacetic acid, followed by hydroxylation.

Process D

The compounds represented by the general formula (III) can be produced by reacting known compounds represented by the general formula (VIII):

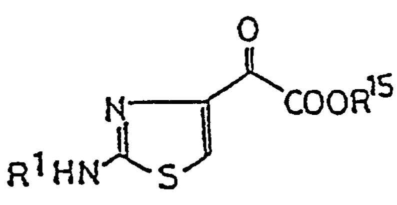

(VIII)

wherein $R^1$ and $R^{15}$ have the same significance as defined above, with compounds represented by the formula (IX):

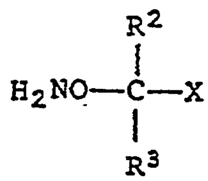

(IX)

wherein $R^2$, $R^3$ and X have the same significance as defined above.

The reaction between the compounds represented by the general formula (VIII) and the compounds represented by the general formula (IX) may be carried out generally in an inert solvent such as alcohols, dioxane, tetrahydrofuran, acetonitrile, chloroform, methylene chloride, ethyl acetate, dimethylformamide, etc. and, if necessary, using suitable dehydrating agents, e.g., molecular sieves.

The reaction may be carried out at temperatures ranging from about 0°C to 50°C, and preferably at from 10°C to 30°C.

If necessary and desired, the protecting groups may be removed from thus obtained aminothiazole acetic acid derivatives represented by the general formula (III).

The compounds represented by the general formula (IX) can be prepared by reacting corresponding halides represented by the general formula (X):

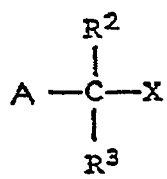

(X)

wherein $R^2$, $R^3$ and X have the same significance as defined above, and A represents a halogen atom, with N-hydroxyphthalimide, followed by the removal of the phthaloyl group with appropriate deprotecting agents such as N-methylhydrazine, hydrazine hydrate, etc.

The aforementioned halides represented by the general formula (X) can be prepared by halogenation of corresponding precursor. For example, α-bromodiacetoxyphenyl acetic acid can be prepared from diacetoxyphenyl acetic acid by conversion into the acid halide and following bromination with N-bromosuccinimide; α-chloro-3,4-carbonyldioxyphenyl acetic acid can be prepared from piperonal by conversion into 3,4-methylenedioxymandelic acid with bromoform followed by phosphorus pentachloride treatment; 4,5-diacetoxy-2-methylbenzoyl chloride can be prepared from 4,5-diacetoxy-2-methylbenzoic acid with thionyl chloride.

Process E

The compounds represented by the general formula (III) can be produced by reacting compounds represented by the general formula (XI):

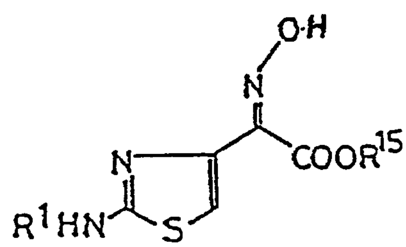

(XI)

wherein $R^1$ and $R^{15}$ have the same significance as defined above, with compounds represented by the general formula (VII):

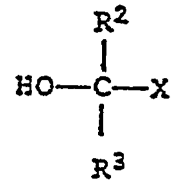

(VII)

wherein $R^2$, $R^3$ and X have the same significance as defined above.

The reaction between the compounds represented by the general formula (XI) and the compounds represented by the general formula (VII) may be carried out in the same manner as described in Process C.

If necessary and desired, the protecting groups may be removed from the thus obtained amino thiazole derivatives represented by the general formula (III).

Process F

The compounds represented by the general formula (III) can be produced by reacting compounds represented by the general formula (XII):

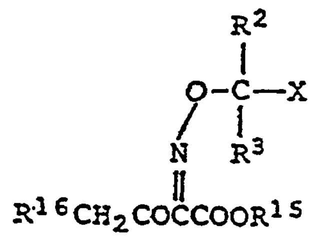

(XII)

wherein $R^2$, $R^3$, $R^{15}$ and X have the same significance as defined above, and $R^{16}$ represents a halogen atom, with compounds represented by the general formula (XIII):

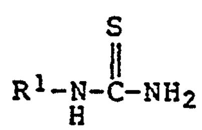

(XIII)

wherein $R^1$ has the same significance as defined above.

The reaction between the compounds represented by the general formula (XII) and the compounds represented by the general formula (XIII) may be carried out generally in an inert solvent such as methanol, ethanol, isopropanol, dioxane, tetrahydrofuran, acetonitrile, ethyl acetate, or dimethylformamide, etc. If necessary, the reaction may be carried out in the presence of deacidifying agents, such as triethylamine, diethylamine, sodium hydrogen carbonate, sodium carbonate, potassium carbonate, etc.

The reaction may be carried out at temperatures ranging from about –10°C to 30°C, and preferably at from –10°C to 10°C.

If necessary and desired, the protecting groups may be removed from thus obtained aminothiazole acetic acid derivatives represented by the general formula (III).

The compounds represented by the general formula (XII) can be prepared by reacting the known compounds represented by the general formula (XIV)

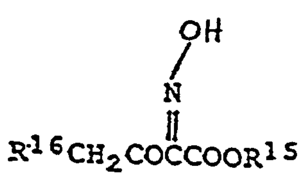

(XIV)

wherein $R^{15}$ and $R^{16}$ have the same significance as defined above, with the compounds represented by the general formula (VII)

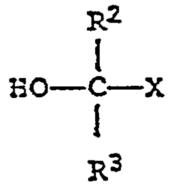

(VII)

wherein $R^2$, $R^3$ and X have the same significance as defined above, in the same manner as described in Process C.

To demonstrate the utility of the compounds of the present invention, data on antibacterial activity of representative compounds are shown below.

Compound 1: (6R,7R)-7-[2-(2-amino-4-thiazolyl)-2-[Z-[(S)-carboxy(3,4-diacetoxyphenyl)methyl]oxy-imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid

Compound 2: (6R,7R)-7-[2-(2-amino-4-thiazolyl)-2-[Z-[(R)-carboxy(3,4-diacetoxyphenyl)methyl]oxy-imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid

Compound 3: (6R,7R)-7-[2-(2-amino-4-thiazolyl)-2-[Z-[(S)-carboxy(3,4-dihydroxyphenyl)methyl]oxy-imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid

Compound 4: (6R,7R)-7-[2-(2-amino-4-thiazolyl)-2-[Z-[(R)-carboxy(3,4-dihydroxyphenyl)methyl]oxy-imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid

Compound 5: (6R,7R)-7-[2-(2-amino-4-thiazolyl)-2-[Z-[1-carboxy-1-(3,4-dihydroxyphenyl)ethyl]oxy-imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid

Experimental Example 1

Antibacterial activity _in vitro_ was determined in accordance with the agar plate dilution method.

A platinum loop each of test bacteria ($10^6$ cells/ml), cultured in Mueller Hinton broth, was inoculated on Mueller Hinton agar plates which contained test compounds at various concentrations. After cultivating at 37°C for 20 hours, the minimum inhibitory concentration (MIC μg/ml) was determined.

The results are shown in Table 1.

Table 1

(MIC μg/ml)

| Compound Number | Staphylococcus aureus Smith | Escherichia coli 67 | Serratia marcescens IFO3759 | Klebsiella pneumoniae IFO3317 | Pseudomonas aeruginosa IFO3445 | Bacteroides fragilis 5524 |
|---|---|---|---|---|---|---|
| 1 | N.D. | 0.20 | 0.10 | <0.05 | 1.56 | N.D. |
| 2 | N.D. | 0.20 | 0.10 | <0.05 | 3.13 | N.D. |
| 3 | 0.78 | 0.10 | 0.05 | <0.05 | 1.56 | 6.25 |
| 4 | 0.78 | 0.20 | 0.10 | <0.05 | 6.25 | N.D. |
| 5 | 0.78 | <0.05 | 0.20 | <0.05 | 0.78 | N.D. |

N.D.; not determined.

Experimental Example 2

Protection ability against systemic infection was determined as follows. An aqueous suspension of test bacteria was intraperitoneally injected into 10 four week old ICR mice. One hour after the infection, test compounds were intravenously administered. The number of surviving mice was counted 1 week after injection to determine the dose at which 50% of the test animals were alive ($ED_{50}$: mg/kg).

The results are shown in Table 2.

Table 2

$ED_{50}$ (mg/Kg)

| Compound Number | Escherichia coli 111 | Serratia marcescenes 274 | Pseudomonas aeruginosa IFO3445 | Staphylococcus aureus 242* |
|---|---|---|---|---|
| 2 | N.D. | 10.5 | N.D. | N.D. |
| 3 | 1.73 | 0.47 | 95.0 | 3.34 |
| 4 | N.D. | 28.6 | N.D. | N.D. |
| CAZ** | 3.91 | 6.41 | 230 | >2100 |
| CMD*** | N.D. | N.D. | N.D. | 8.94 |

N.D.; Not determined.
*; Methicillin-resistant strain
**; Ceftazidime
***; Cefamandole

Next, $LD_{50}$ of representative examples of the compounds of the present invention is shown in Table 3 wherein $LD_{50}$ was determined in accordance with the Probit method.

Table 3

| Compound No. | $LD_{50}$ (mg/Kg, i.v.) |
|---|---|
| 3 | >1000 |

The compounds of the present invention are active against microorganisms, such as Gram-positive aerobic bacteria such as Staphylococcus aureus, streptococci, etc., Gram-negative aerobic bacteria such as Escherichia coli, Klebsiella pneumoniae, Proteus mirabilis, Proteus morganii, Serratia marcescens, Pseudomonas aeruginosa, Citrobacter, Enterobacter, Flavobacter, etc. and anaerobic bacteria such as Peptococci, Peptostreptococci, Bacteroides, etc., and are extremely useful for the treatment of infectious diseases such as brain abscess with Staphylococcus aureus, bacterial meningitis and purulent meningitis with Escherichia coli, Hemophilus influenzae, and Streptococcus pneumoniae, infective endocarditis with Streptococcus epidermidis, Staphylococcus aureus, and Klebsiella pneumoniae, pneumonia with Pseudomonas aeruginosa, Hemophilus influenzae, Klebsiella pneumoniae, and Staphylococcus aureus, and pyelonephritis with Escherichia coli, Klebsiella, Proteus and Pseudomonas etc.

The cephalosporin derivatives provided by the present invention can be employed as pharmaceutical compositions, for example, in the form of pharmaceutical compositions containing cephalosporin derivatives together with appropriate, pharmaceutically acceptable carriers. The pharmaceutical composition may take a solid form (for example, tablets, capsules, etc.) or a liquid form (for example, injections, etc.). The compositions may be sterilized and may contain auxiliary agents generally employed in the pharmaceutical art.

Further, it is preferred to use the compounds after they are formed into freeze-dried products or powders followed by dissolving them in a conventional solvent, e.g., water or physiological saline, for use. The compounds can be used orally or parenterally. While dose varies depending upon age and conditions of the patient, conditions and kind of diseases, etc., from about 0.1 to about 10 g, preferably from about 0.2 to about 5 g, can be used as a daily dose for an adult. Parenteral administration of the compounds provided by the present invention is particularly preferred.

Hereafter the present invention will be described with reference to the examples below but is not deemed to be limited thereof.

Example 1

Preparation of (6R,7R)-7-[2-(2-amino-4-thiazolyl)-2-[Z-[(S)-carboxy(3,4-diacetoxyphenyl)methyl]oxyimino]acetamido]-3-[(2-carboxy-5-methyl-s-triazolo-[1,5-a]pyrimidin-7-yl)thiomethyl]-8-oxo-5-thia-1-azabicyclo[4.2.0]oct-2-ene-2-carboxylic acid (Compound 1).

Step 1

Preparation of 2-bromo-(3,4-diacetoxyphenyl)acetic acid.

Thionyl chloride (60 ml) was added to a suspension of 3,4-diacetoxyphenylacetic acid (51.1 g) in carbon tetrachloride (105 ml) and the mixture was heated at 70°C for one hour. After cooling to room temperature, N-bromosuccinimide (42.3 g), carbon tetrachloride (105 ml) and a little amount of hydrobromic acid were added, and the mixture was heated for an additional one hour. The resulting mixture was concentrated under reduced pressure, and the residue was redissolved in carbon tetrachloride. After filtering off the insoluble matters, the filtrate was dissolved in acetone (400 ml) and the pH of the solution was adjusted to 4.0 with saturated sodium bicarbonate aqueous solution under ice cooling. The resulting mixture was extracted with chloroform. The chloroform layer was washed with brine and dried over anhydrous sodium sulfate. The dried solution was concentrated under reduced pressure, giving 61.4 g of the objective compound.

NMR ($CDCl_3$, δ):
9.0 (1H, brs), 7.5–7.1 (3H, m), 5.3 (1H, s), 2.3 (6H, s)

Step 2

Preparation of 2-bromo-(3,4-diacetoxyphenyl)acetic acid diphenylmethyl ester.

To a solution of the product obtained in Step 1 (61.4 g) in acetone (500 ml) was added diphenyldiazomethane, and the solution was stirred at room temperature for one hour. The resulting solution was concentrated under reduced pressure and the residue was purified by silica gel column chromatography, giving 48.4 g of the objective compound.

IR (KBr, $cm^{-1}$):
1772, 1756, 1752, 1505, 1371, 1259, 1212, 1113, 701
NMR ($CDCl_3$, δ):
7.4–7.1 (13H, m) 6.9 (1H, s), 5.4 (1H, s), 2.3 (6H, s)

Step 3

Preparation of 2-N-phthaloyloxy-(3,4-diacetoxyphenyl)acetic acid diphenylmethyl ester.

To an ice-cooled suspension of N-hydroxyphthalimide (15.9 g) in acetonitrile (300 ml) were added triethylamine (13.6 ml) and a solution of the product obtained in Step 2 (48.4 g) in acetonitrile (200 ml). The mixture was stirred under ice cooling for 1.5 hours. The resulting solution was concentrated under reduced pressure and redissolved in ethyl acetate. The solution was washed with water, 1 N citric acid solution and with brine in that order. The washed solution was dried over anhydrous sodium sulfate and concentrated under reduced pressure, and the residue was purified by silica gel column chromatography, giving 15.3 g of the objective compound.

IR (KBr, $cm^{-1}$):
1772, 1735, 1506, 1371, 1260, 1209, 1186, 1114, 700
NMR ($CDCl_3$, δ):
7.7 (4H, s), 8.0–7.1 (13H, m), 6.9 (1H, s), 2.3 (6H, s)

Step 4

Preparation of 2-aminooxy-(3,4-diacetoxyphenyl)acetic acid diphenylmethyl ester.

To a solution of the product obtained in Step 3 (15.3 g) in dichloromethane (200 ml) was added methylhydrazine (1.34 ml) slowly at −60°C, and the mixture was allowed to stand until room temperature was reached. After stirring for two hours, methylhydrazine (0.07 ml) was added to the mixture, followed by stirring for an additional 30 minutes. The insoluble matters were filtrated off, the filtrate was concentrated under reduced pressure, and the residue was purified by silica gel column chromatography, giving 8.7 g of the objective compound.
IR (KBr, $cm^{-1}$):
1772, 1752, 1506, 1371, 1256, 1210, 1180, 1113, 702
NMR ($CDCl_3$, δ):
7.7–7.0 (13H, m), 6.9 (1H, s), 5.2 (1H, s), 2.27 (3H, s), 2.26 (3H, s)

Step 5

Preparation of 2-(2-triphenylmethylamino-4-thiazolyl)-2-[Z-[diphenylmethyloxycarbonyl(3,4-diacetoxyphenyl)methyl]oxyimino]acetic acid.

To a solution of (2-triphenylmethylamino-4-thiazolyl)glyoxylic acid (7.62 g) in methanol (400 ml) was added dropwise a solution of the product obtained in Step 4 (8.7 g) in methanol (150 ml). The mixture was stirred at room temperature for 1.5 hours, and concentrated under reduced pressure, giving 16.0 g of the objective compound as crude product.
IR (KBr, $cm^{-1}$):
1772, 1256, 1209, 1180, 754, 701
NMR (DMSO-$d_6$, δ):
8.9 (1H, s), 7.8–7.2 (28H, m), 6.9 (1H, s), 6.8 (1H, s), 5.9 (1H, s), 2.3 (6H, s)

Step 6

Preparation of (6R,7R)-7-[2-(2-triphenylmethylamino-4-thiazolyl)-2-[Z-[diphenylmethyloxycarbonyl(3,4-diacetoxyphenyl)methyl]oxyimino]-acetamido]-3-[(2-diphenylmethyloxycarbonyl-5-methyl-s-triazolo[1,5-a]pyrimidin-7-yl)thiomethyl]-8-oxo-5-thia-1-azabicyclo[4.2.0]oct-2-ene-2-carboxylic acid diphenylmethyl ester.

To an ice-cooled solution of the crude product obtained in Step 5 (5.6 g) and (6R,7R)-7-amino-3-[(2-diphenylmethyloxycarbonyl-5-methyl-s-triazolo[1,5-a]pyrimidin-7-yl)thiomethyl]-8-oxo-5-thia-1-azabicyclo[4.2.0]oct-2-ene-2-carboxylic acid diphenylmethyl ester (5.0 g) in dichloromethane (170 ml) was added dicyclohexylcarbodiimide (1.4 g), and the mixture was stirred at room temperature for five hours. After filtering off the insoluble matters, the filtrate was concentrated under reduced pressure. The residue was dissolved in ethyl acetate and the insoluble matters were filtered off. The filtrate was washed with brine and dried over anhydrous sodium sulfate. The dried solution was concentrated under reduced pressure, and the residue was purified by silica gel column chromatography, giving 0.73 g (less polar form) and 1.39 g (more polar form) of the objective compounds.
less polar form
IR (KBr, $cm^{-1}$):
1780, 1742, 1737, 1507, 1249, 1205, 1182, 700
NMR (DMSO-$d_6$, δ):
9.7 (1H, d, J = 8 Hz), 8.9 (1H, brs), 7.5–7.1 (50H, m), 6.9 (1H, s), 6.82 (1H, s), 6.78 (1H, s), 5.9 (1H, s), 5.8 (1H, dd, J = 4, 8 Hz), 5.2 (1H, d, J = 4 Hz), 4.3 (2H, brs), 3.6 (2H, ABq), 2.6 (3H, s), 2.2 (6H, s)
more polar form
IR (KBr, $cm^{-1}$):
1780, 1742, 1596, 1507, 1450, 1372, 1205, 1182, 700
NMR (DMSO-$d_6$, δ):
9.7 (1H, d, J = 9 Hz), 8.9 (1H, s), 7.4–7.2 (50H, m), 7.0 (1H, s), 6.82 (1H, s), 6.76 (1H, s), 5.9 (1H, s), 5.9 (1H, dd, J = 4, 9 Hz), 5.2 (1H, d, J = 4 Hz), 4.3 (2H, brs), 3.7 (2H, ABq), 2.6 (3H, s), 2.20 (6H, s)

Step 7

Preparation of (6R,7R)-7-[2-(2-amino-4-thiazolyl)-2-[Z-[(S)-carboxy(3,4-diacetoxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

To a solution of the less polar form of the product obtained in Step 6 (0.73 g) in dichloroethane (3 ml) were added anisole (0.4 ml) and trifluoroacetic acid (0.8 ml) under ice cooling, and the resulting solution was stirred at room temperature for three hours. Additional trifluoroacetic acid (0.6 ml) was added and the mixture was stirred for another 30 minutes. After removing the solvent by decantation, the residue was washed with dichloroethane and crystallized with ether, giving 0.3 g of the objective compound (as trifluoroacetic acid salt).
IR (KBr, $cm^{-1}$):
1773, 1735, 1684, 1637, 1598, 1509, 1373, 1206, 1186
NMR (DMSO-$d_6$, δ):
9.6 (1H, d, J = 8 Hz), 7.6–7.2 (4H, m), 6.8 (1H, s), 5.8 (1H, dd, J = 4, 8 Hz), 5.6 (1H, s), 5.2 (1H, d, J = 4 Hz), 4.4 (2H, brs), 3.72 (1H, d, J = 22 Hz), 3.48 (1H, d, 22 Hz), 2.6 (3H, s), 2.2 (6H, s)
$[\alpha]^{25}$ −2.9° (c = 1.0, methanol:acetone = 1:1)

Example 2

Preparation of (6R,7R)-7-[2-(2-amino-4-thiazolyl)-2-[Z-[(S)-carboxy(3,4-dihydroxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid (Compound 3).

The product obtained in Step 7 of Example 1 (0.27 g) was suspended in water (11 ml), and the pH of the mixture was adjusted to 8.0 with sodium bicarbonate. After stirring at room temperature for six hours, the resulting solution was applied to a Diaion HP 10 column. The objective fractions eluted with water were collected and lyophilized, giving 0.14 g of the objective compound (as sodium salt).
IR (KBr, $cm^{-1}$):
1763, 1599, 1514, 1474, 1404, 1360, 1314
NMR ($D_2O$, δ):
7.2 (1H, s), 7.0–6.8 (4H, m), 5.7 (1H, d, J = 5 Hz), 5.4 (1H, s), 5.0 (1H, d, J = 5 Hz), 4.3 (2H, ABq), 3.4 (2H, ABq), 2.6 (3H, s)
$[\alpha]^{25}$ +27.4° (c = 1.0, water)

Example 3

Preparation of (6R,7R)-7-[2-(2-amino-4-thiazolyl)-2-[Z-[(R)-carboxy(3,4-dihydroxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid (Compound 4).

Step 1

Preparation of (6R,7R)-7-[2-(2-amino-4-thiazolyl)-2-[Z-[(R)-carboxy(3,4-diacetoxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

To a solution of the more polar form of the product obtained in Step 6 of Example 1 (1.3 g) in dichloroethane (6 ml) were added anisole (0.8 ml) and trifluoroacetic acid (1.6 ml) under ice cooling, and the resulting solution was stirred at room temperature for 4 hours. After addition of dichloroethane (6 ml), the solvent was removed by decantation. The residue was washed with dichloroethane, and crystallized with ether, giving 0.78 g of the objective compound (as trifluoroacetic acid salt).
IR (KBr, $cm^{-1}$):
1773, 1735, 1683, 1636, 1598, 1509, 1373, 1205, 1185
NMR (DMSO-$d_6$, δ):
9.7 (1H, d, J = 9 Hz), 7.4–7.2 (4H, m), 6.8 (1H, s), 5.8 (1H, dd, J = 4, 9 Hz), 5.6 (1H, s), 5.2 (1H, d, J = 4 Hz), 4.5 (2H, brs), 3.79 (1H, d, J = 17 Hz), 3.60 (1H, d, J = 17 Hz), 2.6 (3H, s), 2.3 (6H, s)
$[\alpha]^{25}$ −17.4° (c = 1.0, methanol:acetone = 1:1)

Step 2

Preparation of (6R,7R)-7-[2-(2-amino-4-thiazolyl)-2-[Z-[(R)-carboxy(3,4-dihydroxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

The product obtained in Step 1 (0.5 g) was suspended in water (20 ml), and the pH of the mixture was adjusted to 7.6–8.0 with sodium bicarbonate. After stirring at room temperature for six hours, the resulting solution was applied to a Diaion HP 10 column. The objective fractions eluted with water were collected and lyophilized, giving 0.2 g of the objective compound (as sodium salt).
IR (KBr, $cm^{-1}$):
1763, 1601, 1516, 1474, 1404, 1358, 1314
NMR ($D_2O$, δ):
7.2 (1H, s), 7.0–6.9 (4H, m), 5.6 (1H, d, J = 5 Hz), 5.4 (1H, s), 5.0 (1H, d, J = 5 Hz), 4.4 (2H, ABq), 3.4 (2H, ABq), 2.6 (3H, s)
$[\alpha]^{25}$ +21.8° (c = 1.0, water)

Example 4

Preparation of (6R,7R)-7-[2-(2-amino-4-thiazolyl)-2-[Z-[carboxy(3,4-dihydroxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

Step 1

Preparation of 2-(2-amino-4-thiazolyl)-2-[Z-[diphenylmethyloxycarbonyl(3,4-diacetoxyphenyl)methyl]-oxyimino]acetic acid.

To an ice-cooled solution of product obtained in Step 4 of Example 1 (5.3 g) in dimethylformamide (18 ml) was added (2-aminothiazol-4-yl)glyoxylic acid (2.03 g), and the mixture was stirred overnight at room temperature. The resulting solution was poured into ice water (100 ml), and the mixture was acidified (pH 2) with 1 N hydrochloric acid and extracted with ethyl acetate. The organic layer was washed with brine and dried over anhydrous magnesium sulfate. The dried solution was concentrated under reduced pressure, and the residue was crystallized with ether, giving 6.30 g of the objective compound.
NMR (DMSO-$d_6$, δ):
7.5–7.2 (15H, m), 6.85 (1H, s), 6.83 (1H, s), 5.9 (1H, s), 2.3 (6H, s)

Step 2

Preparation of (6R,7R)-7-[2-(2-amino-4-thiazolyl)-2-[Z-[diphenylmethyloxycarbonyl(3,4-diacetoxyphenyl)methyl]oxyimino]acetamido]-3-[(2-diphenylmethyloxycarbonyl-5-methyl-s-triazolo[1,5-a]-pyrimidin-7-yl)thiomethyl]-8-oxo-5-thia-1-azabicyclo[4.2.0]oct-2-ene-2-carboxylic acid diphenylmethyl ester.

To an ice-cooled solution containing the product obtained in Step 1 (3.0 g) and (6R,7R)-7-amino-3-[(2-diphenylmethyloxycarbonyl-5-methyl-s-triazolo[1,5-a]pyrimidin-7-yl)thiomethyl]-8-oxo-5-thia-1-azabicyclo[4.2.0]oct-2-ene-2-carboxylic acid diphenylmethyl ester (3.75 g) in dichloromethane (100 ml) was added dicyclohexylcarbodiimide (1.54 g), and the mixture was stirred at room temperature. After filtering off the insoluble matters, the filtrate was concentrated under reduced pressure, and the residue was purified by silica gel column chromatography, giving 4.8 g of the objective compound.
NMR (DMSO-$d_6$, δ):
9.9 and 9.8 (1H, d, J = 8 Hz), 7.4–6.8 (38H, m), 5.9 (1H, m), 5.9 (1H, s), 5.3 and 5.2 (1H, d, J = 5 Hz), 4.3 (2H, brs), 3.6 (2H, ABq), 2.6 (3H, s), 2.3 (3H, s), 2.2 (3H, s)

Step 3

Preparation of (6R,7R)-7-[2-(2-amino-4-thiazolyl)-2-[Z-[carboxy(3,4-diacetoxyphenyl)methyl]oxyimino]-acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

To a solution of the product obtained in Step 2 (0.87 g) in dichloroethane (1.6 ml) were added anisole (0.8 ml) and trifluoroacetic acid (2.4 ml) under ice cooling, and the resulting solution was stirred at room temperature for two hours. After removing the solvent by decantation, the residue was washed with dichloroethane and crystallized with ether, giving 0.6 g of the objective compound (as trifluoroacetic acid salt).

NMR (DMSO-$d_6$, δ):
9.8 and 9.6 (1H, d, J = 8 Hz), 7.4–6.9 (4H, m), 6.83 and 6.79 (1H, s), 5.8 (1H, m), 5.6 (1H, s), 5.2 (1H, m), 4.4 (2H, brs), 3.7 (2H, ABq), 2.6 (3H, s), 2.26 (3H, s), 2.24 (3H, s)

Step 4

Preparation of (6R,7R)-7-[2-(2-amino-4-thiazolyl)-2-[Z-[carboxy(3,4-dihydroxyphenyl)methyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

The product obtained in Step 3 (0.25 g) was suspended in water (10 ml), and the pH of the mixture was adjusted to 8.0 with sodium bicarbonate. After stirring at room temperature for 6 hours, the resulting solution was applied to a Diaion HP 10 column. The objective fractions eluted with water were collected and lyophilized, giving 0.14 g of the objective compound (as sodium salt).
NMR ($D_2O$, δ):
7.2–6.9 (5H, m), 5.7 (1H, m), 5.4 (1H, s), 5.0 (1H, m), 4.3 (2H, ABq), 3.4 (2H, ABq), 2.6 (3H, s)

Example 5

Preparation of (6R,7R)-7-[2-(2-amino-4-thiazolyl)-2-[Z-[1-carboxy-1-(3,4-dihydroxyphenyl)ethyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid (Compound 5).

Step 1

Preparation of α-bromo-α-methyl-3,4-diacetoxyphenylacetic acid diphenylmethyl ester.

To a suspension of α-methyl-3,4-diacetoxyphenyl acetic acid (10.0 g) in carbon tetrachloride (10 ml) were added thionyl chloride (12 ml) and a little amount of dimethylformamide, and the mixture was stirred at 70°C for 30 minutes. The solvent was removed under reduced pressure, and the residue was redissolved in carbon tetrachloride (20 ml). Thionyl chloride (5 ml), N-bromosuccinimide (7.22 g) and hydrobromic acid (0.1 ml) were added to the solution, and the mixture was stirred at 85°C for 1.5 hours. After filtering off the insoluble matters, the filtrate was concentrated under reduced pressure. The residue was dissolved in acetone (60 ml), and the pH of the solution was adjusted to 5 with saturated sodium bicarbonate solution under ice cooling, then to 1 with 1 N hydrochloric acid. The acidified mixture was extracted with ethyl acetate (400 ml), and the extract was washed with brine and dried over anhydrous sodium sulfate. The dried solution was concentrated under reduced pressure, and the residue was dissolved in acetone (60 ml) and diphenyldiazomethane (7.0 g) was added. The solution was stirred overnight and the resulting solution was concentrated under reduced pressure. The residue was purified by silica gel column chromatography, giving 4.1 g of the objective compound.
NMR ($CDCl_3$, δ):
7.4–7.0 (13H, m), 6.9 (1H, s), 2.28 (6H, s), 2.27 (3H, s)

Step 2

Preparation of α-methyl-α-phthaloyloxy-3,4-diacetoxyphenylacetic acid diphenylmethyl ester.

To an ice-cooled solution of the product obtained in Step 1 (4.1 g) were added N-hydroxyphthalimide (1.31 g) and then anhydrous potassium carbonate over a period of 10 minutes. After stirring at room temperature for 1.5 hours, the resulting solution was poured into 1 N citric acid aqueous solution (100 ml) and extracted with ethyl acetate (100 ml). The extract was washed thrice with brine and dried over anhydrous sodium sulfate. After concentrating under reduced pressure, the residue was purified by silica gel column chromatography, giving 1.3 g of the objective compound.
IR (KBr, $cm^{-1}$):
1773, 1741, 1736, 1372, 1263, 1208, 1191, 1170, 1119, 702
NMR ($CDCl_3$, δ):
7.8 (4H, m), 7.4–7.2 (13H, m), 6.9 (1H, s), 2.28 (3H, s), 2.27 (3H, s), 1.9 (3H, s)

Step 3

Preparation of α-aminooxy-α-methyl-3.4-diacetoxyphenylacetic acid diphenylmethyl ester.

To a solution of the product obtained in Step 2 (1.3 g) in dry dichloromethane (20 ml) was added methylhydrazine (0.2 g) at –70°C under a nitrogen stream, and the solution was stirred at –70°C for 10 minutes and then at 0°C for 40 minutes. After filtering off the insoluble matters, the filtrate was concentrated under reduced pressure, and the residue was purified by silica gel column chromatography, giving 0.61 g of the objective compound.
NMR ($CDCl_3$, δ):
7.3–7.0 (13H, m), 6.9 (1H, s), 2.28 (3H, s), 2.26 (3H, s), 1.9 (3H, s)

Step 4

Preparation of 2-(2-triphenylmethylamino-4-thiazolyl)-2-[Z-[1-diphenylmethyloxycarbonyl-1-(3,4-diacetoxyphenyl)ethyl]oxyimino]acetic acid.

To a solution of (2-triphenylmethylamino-4-thiazolyl)glyoxylic acid (0.49 g) in methanol (25 ml) was added dropwise a solution of the product obtained in Step 3 (0.61 g) in methanol (10 ml). The mixture was stirred at room temperature for 1.5 hours and concentrated under reduced pressure. The residue was purified by silica gel column chromatography, giving 0.8 g of the objective compound.
IR (KBr, $cm^{-1}$):
1773, 1751, 1743, 1262, 1209, 1168, 1115, 701
NMR (DMSO-$d_6$, δ):
8.8 (1H, s), 7.3–7.1 (28H, m), 6.8 (1H, s), 6.7 (1H, s), 2.3 (6H, s), 1.9 (3H, s)

Step 5

Preparation of (6R,7R)-7-[2-(2-triphenylmethylamino-4-thiazolyl)-2-[Z-[1-diphenylmethyloxycarbonyl-1-(3,4-diacetoxyphenyl)ethyl]oxyimino]acetamido]-3-[(2-diphenylmethyloxycarbonyl-5-methyl-s-triazolo-[1,5-a]pyrimidin-7-yl)thiomethyl]-8-oxo-5-thia-1-azabicyclo[4.2.0]oct-2-ene-2-carboxylic acid diphenylmethyl ester.

To an ice-cooled solution of the product obtained in Step 4 (0.8 g) and (6R,7R)-7-amino-3-[(2-diphenylmethyloxycarbonyl-5-methyl-s-triazolo[1,5-a]pyrimidin-7-yl)thiomethyl]-8-oxo-5-thia-1-azabicyclo-[4.2.0]oct-2-ene-2-carboxylic acid diphenylmethyl ester (0.7 g) in dichloromethane (30 ml) was added dicyclohexylcarbodiimide (0.19 g), and the mixture was stirred overnight at room temperature. After filtering off the insoluble matters, the filtrate was concentrated under reduced pressure, and the residue was purified by silica gel column chromatography, giving 0.6 g of the objective compound.
IR (KBr, $cm^{-1}$):
1791, 1774, 1741, 1736, 1507, 1207, 1171, 700
NMR (DMSO-$d_6$, δ):
9.9 and 9.7 (1H, d, J = 8 Hz), 8.9 (1H, s), 7.5–6.8 (53H, m), 5.9–5.7 (1H, m), 5.2 (1H, d, J = 5 Hz), 4.3 (2H, brs), 3.7 (2H, ABq), 2.6 (3H, s), 2.23 (3H, s), 2.19 (3H, s), 1.9 (3H, s)

Step 6

Preparation of (6R,7R)-7-[2-(2-amino-4-thiazolyl)-2-[Z-[1-carboxy-1-(3,4-diacetoxyphenyl)ethyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

To a solution of the product obtained in Step 5 (0.6 g) in dichloroethane (1 ml) were added anisole (0.5 ml) and trifluoroacetic acid (1 ml) under ice cooling, and the mixture was stirred at room temperature for 2 hours. Trifluoroacetic acid (1 ml) was added again and the mixture was stirred overnight at room temperature. After addition of dichloroethane (20 ml) to the resulting solution, the solvent was removed by decantation, and the residue was crystallized with ether, giving 0.31 g of the objective compound (as trifluoroacetic acid salt).
IR (KBr, $cm^{-1}$):
1772, 1735, 1683, 1636, 1597, 1509, 1263, 1232, 1203, 1172
NMR (DMSO-$d_6$, δ):
9.8–9.7 (1H, m), 7.4–7.0 (4H, m), 6.78 and 6.74 (1H, s), 5.8–5.7 (1H, m), 5.3–5.2 (1H, m), 4.4 (2H, brs), 3.7–3.6 (2H, m), 2.6 (3H, s), 2.2 (6H, s), 1.8 (3H, brs)

Step 7

Preparation of (6R,7R)-7-[2-(2-amino-4-thiazolyl)-2-[Z-[1-carboxy-1-(3,4-dihydroxyphenyl)ethyl]oxyimino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid.

The product obtained in Step 6 (0.28 g) was suspended in water (11 ml), and the pH of the mixture was adjusted to 8.5 with sodium bicarbonate. The mixture was stirred at room temperature for 5.5 hours, and

the resulting solution was applied to a Diaion HP 10 column. The objective fractions were collected and lyophilized, giving 0.094 g of the objective compound (as sodium salt).
IR (KBr, $cm^{-1}$):
1772, 1596, 1509, 1404, 1395, 1389, 1355, 1311
NMR ($D_2O$, δ):
7.2–6.8 (5H, m), 5.8–5.7 (1H, m), 5.2–5.1 (1H, m), 4.5 (2H, ABq), 3.5 (2H, ABq), 2.6 (3H, s), 1.8 (3H, s)

Table 4 provides a structure of Compound 6 together with its spectral data. This compound has been prepared by a reaction procedure similar to Example 1.

Table 4

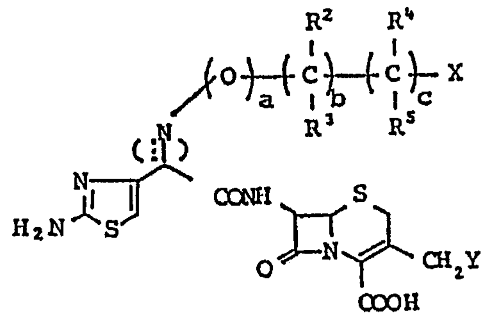

| Example No. | X | Y | $R^2$ | $R^3$ | $R^4$ | $R^5$ | a | b | c | a bond of (¦¦) | N M R , I R |
|---|---|---|---|---|---|---|---|---|---|---|---|
| 6 (TFA* salt) | 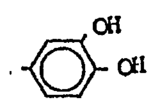 | 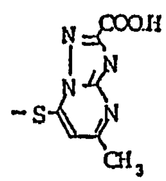 | H | H | — | — | 1 | 1 | 0 | double bond | NMR (δ, DMSO-$d_6$); 9.8 (1H, d; J = 8 Hz), 7.4 - 6.7 (5H, m), 5.8 (1H, dd, J = 5, 8 Hz), 5.2 (1H, d, J = 5 Hz), 4.9 (2H, brs), 4.4 (2H, brs), 3.7 (2H, ABq), 2.6 (3H, s). IR ($cm^{-1}$); 1780, 1683, 1637, 1599, 1509, 1198 |

The following example A details a typical pharmaceutical preparation containing the cephalosporin derivatives of the present invention. This example is not intended to limit the types of compounds to be used, but the method is applicable to all the compounds of the present invention.

Example A (Method of manufacturing freeze-dried parenteral injections)

(6R,7R)-7-[2-(2-amino-4-thiazolyl)-2-[Z-carboxy-[(3,4-dihydroxyphenyl)methyl]imino]acetamido]-3-[(2-carboxy-5-methyl-s-triazolo[1,5-a]pyrimidin-7-yl)thiomethyl]-8-oxo-5-thia-1-azabicyclo[4.2.0]oct-2-ene-2-carboxylic acid (1.1 g) was dissolved in 22 ml of sterile water containing an equivalent amount of sodium bicarbonate, and 2 ml each of this solution was poured into 5-ml ampoules, freeze-dried and sealed by ordinary methods, to produce a freeze-dried preparation for parenteral injections.

**Claims for the Contracting States: BE, CH, DE, FR, GB, IT, LI, LU, NL, SE**

1. A cephalosporin derivative represented by the general formula (I):

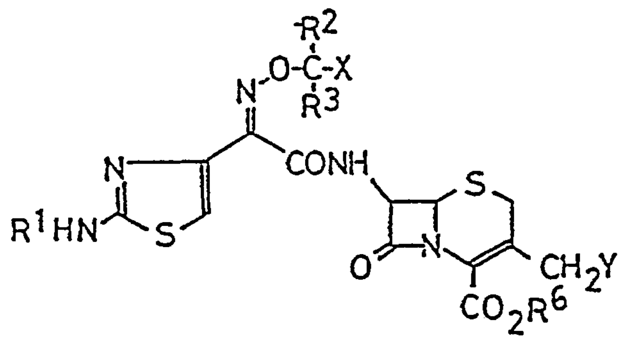

(I)

or a pharmaceutically or veterinarily acceptable non-toxic salt, or solvate, or a non-toxic salt of a solvate thereof; wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^6$ represents a hydrogen atom or a conventional carboxyl protecting group, X represents a group:

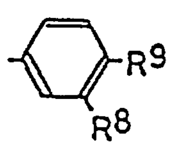

wherein $R^8$ and $R^9$ are the same or different and represent hydroxy groups or acetoxy groups, and Y represents a group:

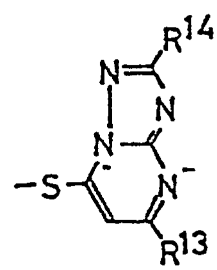

wherein $R^{13}$ represents a methyl group, and $R^{14}$ represents a carboxyl group or a conventionally protected carboxyl group.

2. A cephalosporin derivative according to claim 1, wherein $R^1$ and $R^6$ represent hydrogen atoms.

3. A cephalosporin derivative according to claim 1 or claim 2, wherein $R^2$ represents a carboxyl group or a conventionally protected carboxyl group.

4. A cephalosporin derivative according to any preceding claim, wherein $R^2$ represents a carboxyl group, $R^3$ represents a hydrogen atom, $R^8$ and $R^9$ represent hydroxy groups, $R^{13}$ represents a methyl group, and $R^{14}$ represents a carboxyl group.

5. A cephalosporin derivative according to any preceding claim wherein the absolute configuration of the carbon atom to which $R^2$ and $R^3$ are connected is (S)-configuration.

6. A pharmaceutical composition which comprises a pharmaceutically or veterinarily acceptable carrier and at least one cephalosporin derivative according to any of claims 1–5.

7. A pharmaceutical composition according to claim 6 comprising a pharmaceutically or veterinarily effective amount of the at least one cephalosporin derivative.

8. A process for producing a cephalosporin derivative according to any of claims 1–5 comprising reacting a compound represented by the formula (III):

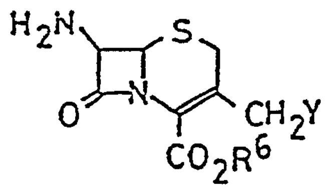

(III)

wherein $R^6$ represents a hydrogen atom or conventional carboxyl-protecting group, and Y represents a group:

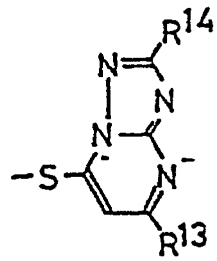

wherein $R^{13}$ represents a methyl group, and $R^{14}$ represents a carboxyl group, or a conventionally protected carboxyl group, or a salt thereof with a compound represented by the formula (IV):

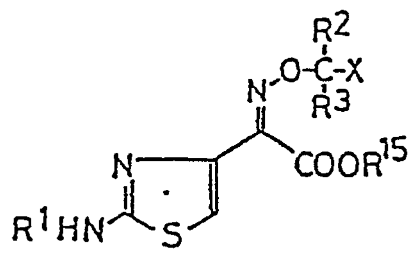

(IV)

wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^{15}$ represents a hydrogen atom or a conventional carboxyl-protecting group, and X represents a group:

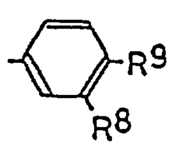

wherein $R^8$ and $R^9$ are the same or different and represent hydroxy groups or acetoxy groups.

9. A process for producing a cephalosporin derivative according to any of claims 1–5 comprising reacting a compound represented by the formula (V):

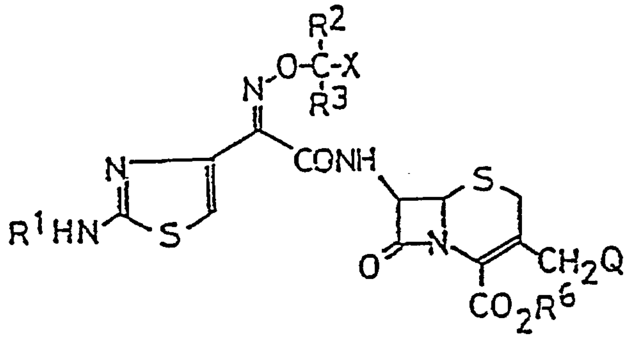 (V)

wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^6$ represents a hydrogen atom or a conventional carboxyl-protecting group, Q represents a chlorine atom, a bromine atom, an iodine atom or an acetoxy group, and X represents a group:

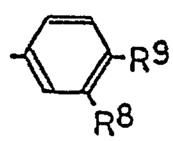

wherein $R^8$ and $R^9$ are the same or different and represent hydroxy groups or acetoxy groups, with a compound represented by the formula (VI):

H–Y (VI)

wherein H represents a hydrogen atom, and Y represents a group:

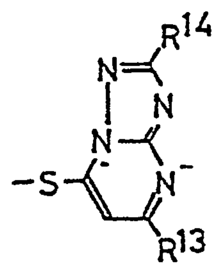

wherein $R^{13}$ represents a methyl group, and $R^{14}$ represents a carboxyl group or a conventionally protected carboxyl group.

10. A process for producing a cephalosporin compound according to any of claims 1–5 comprising reacting a compound represented by the formula (VII):

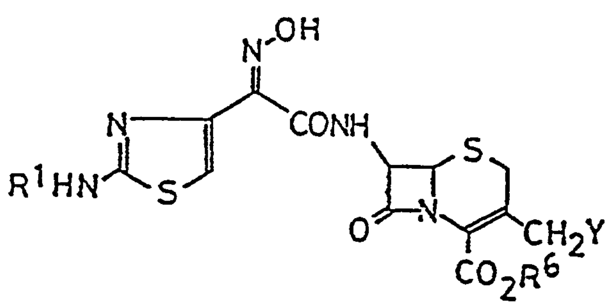 (VII)

wherein $R^1$ represents a hydrogen atom or a conventional amino-protecting group, $R^6$ represents a hydrogen atom or a conventional carboxyl-protecting group, Y represents a group:

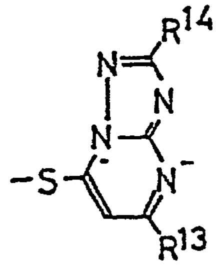

wherein $R^{13}$ represents a methyl group, and $R^{14}$ represents a carboxyl group, a conventionally protected carboxyl group, or a salt thereof with a compound represented by the formula (VIII):

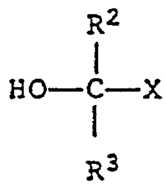

(VIII)

wherein $R^2$ represents a hydrogen atom, a methyl group, carboxyl group, or a conventionally protected carboxyl group, $R^3$ represents a hydrogen atom or a methyl group, and X represents a group:

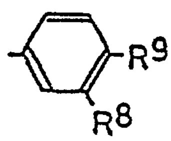

wherein $R^8$ and $R^9$ are the same or different and represent hydroxy groups or acetoxy groups.

11. The use of a cephalosporin derivative according to any of claims 1–5 for the manufacture of an antibacterial agent.

**Claims for the Contracting State: AT**

1. The use of a cephalosporin derivative represented by the general formula (I):

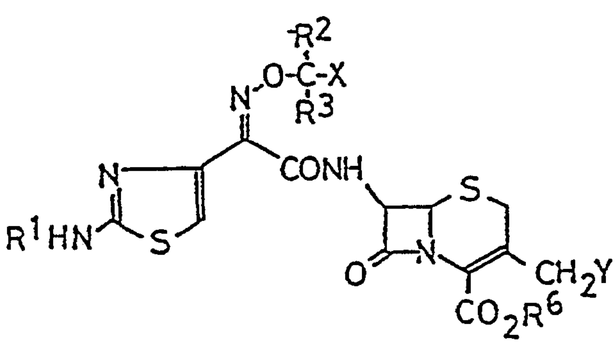

(I)

or a pharmaceutically or veterinarily acceptable non-toxic salt, or solvate, or a non-toxic salt of a solvate thereof; wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^6$ represents a hydrogen atom or a conventional carboxyl protecting group, X represents a group:

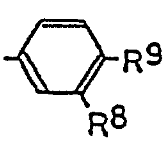

wherein $R^8$ and $R^9$ are the same or different and represent hydroxy groups or acetoxy groups, and Y represents a group:

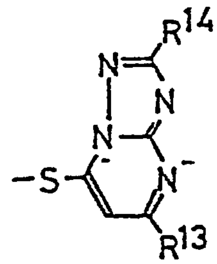

wherein $R^{13}$ represents a methyl group, and $R^{14}$ represents a carboxyl group or a conventionally protected carboxyl group, for the manufacture of an antibacterial agent.

2. The use according to claim 1, wherein $R^1$ and $R^6$ represent hydrogen atoms.

3. The use according to claim 1 or claim 2, wherein $R^2$ represents a carboxyl group or a conventionally protected carboxyl group.

4. The use according to any preceding claim, wherein $R^2$ represents a carboxyl group, $R^3$ represents a hydrogen atom, $R^8$ and $R^9$ represent hydroxy groups, $R^{13}$ represents a methyl group, and $R^{14}$ represents a carboxyl group.

5. The use according to any preceding claim, wherein the absolute configuration of the carbon atom to which $R^2$ and $R^3$ are connected is (S)-configuration.

6. A process for producing a cephalosporin derivative represented by the general formula (I):

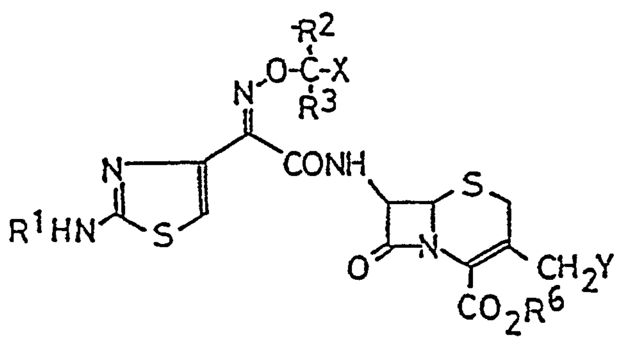

(I)

or a pharmaceutically or veterinarily acceptable non-toxic salt, or solvate or a non-toxic salt of a solvate thereof; wherein $R^1$, $R^2$, $R^3$, $R^6$, X and Y are as defined in claim 1 comprising reacting a compound represented by the formula (III):

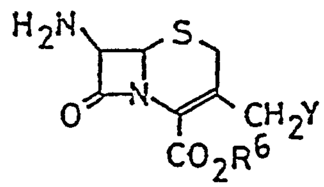

(III)

wherein $R^6$ represents a hydrogen atom or conventional carboxyl-protecting group, and Y represents a group:

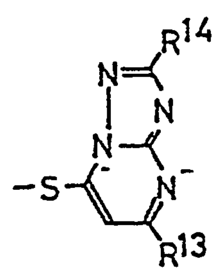

wherein $R^{13}$ represents a methyl group, and $R^{14}$ represents a carboxyl group, or a conventionally protected carboxyl group, or a salt thereof with a compound represented by the formula (IV):

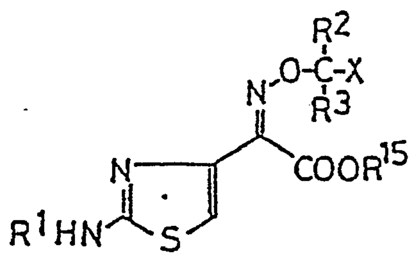

(IV)

wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^{15}$ represents a hydrogen atom or a conventional carboxyl-protecting group, and X represents a group:

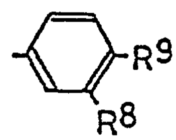

wherein $R^8$ and $R^9$ are the same or different and represent hydroxy groups or acetoxy groups.

7. A process for producing a cephalosporin derivative represented by the general formula (I):

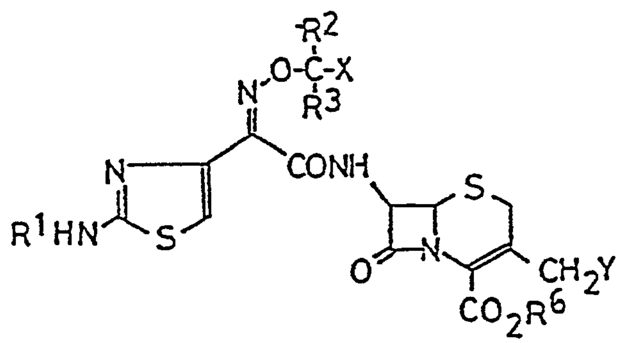

(I)

or a pharmaceutically or veterinarily acceptable non-toxic salt, or solvate or a non-toxic salt of a solvate thereof; wherein $R^1$, $R^2$, $R^3$, $R^6$, X and Y are as defined in claim 1 comprising reacting a compound represented by the formula (V):

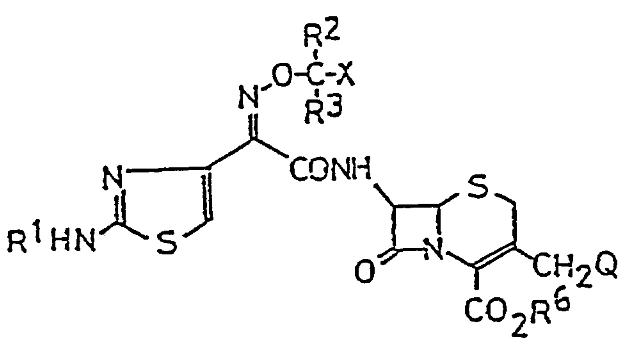

(V)

wherein $R^1$ represents a hydrogen atom or a conventional amino-protecting group, $R^2$ represents a hydrogen atom, a methyl group, a carboxyl group, or a conventionally protected carboxyl group, $R^3$ represents a hydrogen atom or a methyl group, $R^6$ represents a hydrogen atom or a conventional carboxyl-protecting group, Q represents a chlorine atom, a bromine atom, an iodine atom or an acetoxy group, and X represents a group:

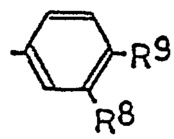

wherein $R^8$ and $R^9$ are the same or different and represent hydroxy groups or acetoxy groups, with a compound represented by the formula (VI):

H–Y (VI)

wherein H represents a hydrogen atom, and Y represents a group:

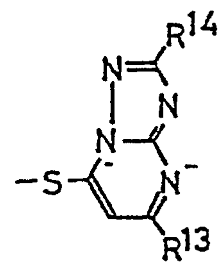

wherein $R^{13}$ represents a methyl group, and $R^{14}$ represents a carboxyl group or a conventionally protected carboxyl group.

8. A process for producing a cephalosporin derivative represented by the general formula (I):

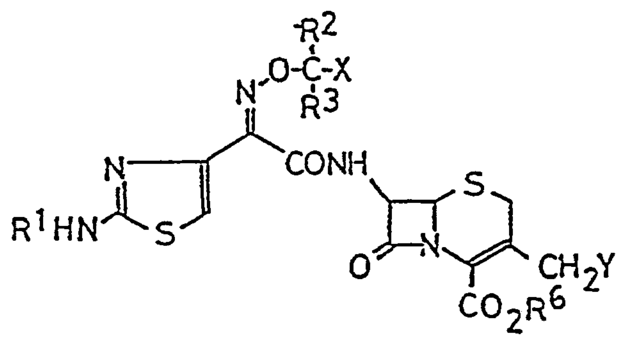

(I)

or a pharmaceutically or veterinarily acceptable non-toxic salt, or solvate or a non-toxic salt of a solvate thereof; wherein $R^1$, $R^2$, $R^3$, $R^6$, X and Y are as defined in claim 1 comprising reacting a compound represented by the formula (VII):

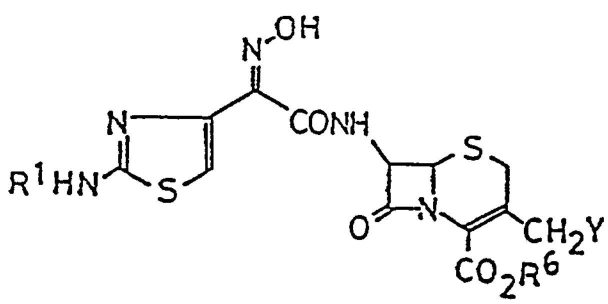

(VII)

wherein $R^1$ represents a hydrogen atom or a conventional amino-protecting group, $R^6$ represents a hydrogen atom or a conventional carboxyl-protecting group, Y represents a group:

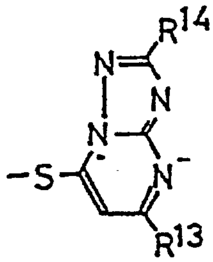

wherein $R^{13}$ represents a methyl group, and $R^{14}$ represents a carboxyl group, a conventionally protected carboxyl group, or a salt thereof with a compound represented by the formula (VIII):

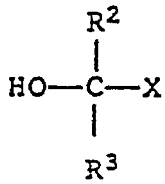

(VIII)

wherein $R^2$ represents a hydrogen atom, a methyl group, carboxyl group, or a conventionally protected carboxyl group, $R^3$ represents a hydrogen atom or a methyl group, and X represents a group:

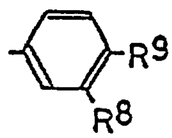

wherein $R^8$ and $R^9$ are the same or different and represent hydroxy groups or acetoxy groups.

9. A cephalosporin derivative represented by the general formula (I):

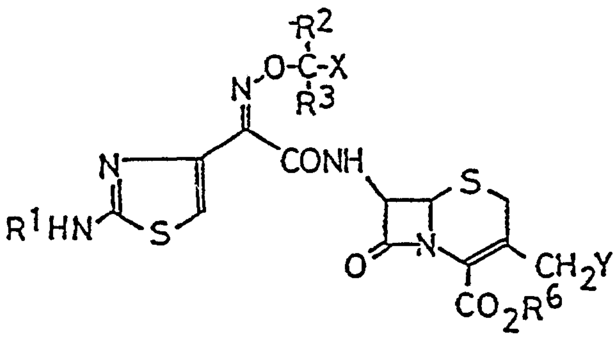

(I)

or a pharmaceutically or veterinarily acceptable non-toxic salt, or solvate, or a non-toxic salt of a solvate thereof; wherein $R^1$, $R^2$, $R^3$, $R^6$, X and Y are as defined in claim 1.

10. A pharmaceutical composition which comprises a pharmaceutically or veterinarily acceptable carrier and at least one cephalosporin derivative according to claim 9.

11. A pharmaceutical composition according to claim 10 comprising a pharmaceutically or veterinarily effective amount of the at least one cephalosporin derivative.

**Patentansprüche für die Vertragsstaaten: BE, CH, LI, DE, FR, GB, IT, LU, NL, SE**

1. Cephalosporinderivat der allgemeinen Formel (I)

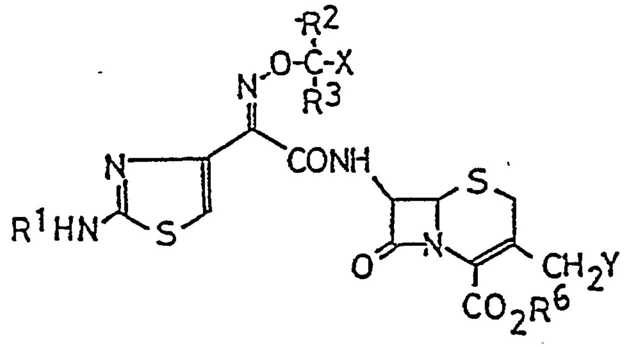

(I)

oder ein pharmazeutisch oder veterinär-medizinisch annehmbares, nicht-toxisches Salz, oder Solvat, oder ein nicht-toxisches Salz eines Solvats davon; worin $R^1$ ein Wasserstoffatom oder eine konventionelle Aminoschutzgruppe bedeutet, $R^2$ ein Wasserstoffatom, eine Methylgruppe, eine Carboxylgruppe, oder eine konventionell geschützte Carboxylgruppe bedeutet, $R^3$ ein Wasserstoffatom oder eine Methylgruppe bedeutet, $R^6$ ein Wasserstoffatom oder eine konventionelle Carboxylschutzgruppe bedeutet, X eine Gruppe

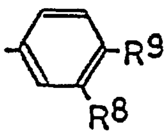

bedeutet, worin $R^8$ und $R^9$ gleich oder verschieden sind und Hydroxygruppen oder Acetoxygruppen bedeuten, und Y eine Gruppe

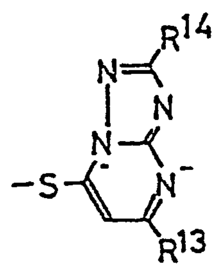

bedeutet, worin $R^{13}$ eine Methylgruppe bedeutet und $R^{14}$ eine Carboxylgruppe oder eine konventionell geschützte Carboxylgruppe bedeutet.

2. Cephalosporinderivat nach Anspruch 1, dadurch gekennzeichnet, daß $R^1$ und $R^6$ Wasserstoffatome bedeuten.

3. Cephalosporinderivat nach Anspruch 1 oder Anspruch 2, dadurch gekennzeichnet, daß $R^2$ eine Carboxylgruppe oder eine konventionell geschützte Carboxylgruppe bedeutet.

4. Cephalosporinderivat nach einem der vorhergehenden Ansprüche, dadurch gekennzeichnet, daß $R^2$ eine Carboxylgruppe bedeutet, $R^3$ ein Wasserstoffatom bedeutet, $R^8$ und $R^9$ Hydroxygruppen bedeuten, $R^{13}$ eine Methylgruppe bedeutet und $R^{14}$ eine Carboxylgruppe bedeutet.

5. Cephalosporinderivat nach einem der vorhergehenden Ansprüche, dadurch gekennzeichnet, daß die absolute Konfiguration des Kohlenstoffatoms, an das $R^2$ und $R^3$ gebunden sind, die (S)-Konfiguration ist.

6. Pharmazeutische Zusammensetzung umfassend einen pharmazeutisch oder veterinär-medizinisch annehmbaren Träger und mindestens ein Cephalosporinderivat nach einem der Ansprüche 1 bis 5.

7. Pharmazeutische Zusammensetzung nach Anspruch 6, dadurch gekennzeichnet, daß sie eine pharmazeutisch oder veterinär-medizinisch wirksame Menge von mindestens einem Cephalosporinderivat enthält.

8. Verfahren zur Herstellung eines Cephalosporinderivats nach einem der Ansprüche 1 bis 5, dadurch gekennzeichnet, daß man eine Verbindung der allgemeinen Formel (III)

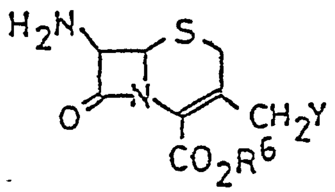

(III)

worin $R^6$ ein Wasserstoffatom oder eine konventionelle Carboxylschutzgruppe bedeutet, und Y eine Gruppe

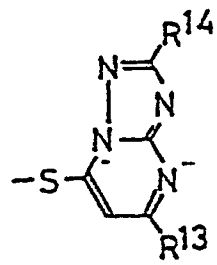

bedeutet, worin $R^{13}$ eine Methylgruppe bedeutet und $R^{14}$ eine Carboxylgruppe oder eine konventionell geschützte Carboxylgruppe bedeutet, oder ein Salz davon, mit einer Verbindung der Formel (IV)

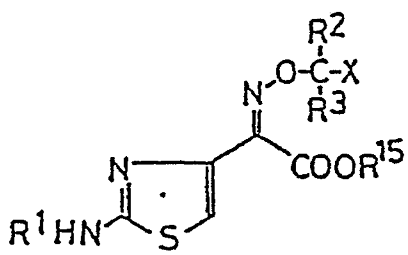

(IV)

worin $R^1$ ein Wasserstoff oder eine konventionelle Aminoschutzgruppe bedeutet, $R^2$ ein Wasserstoffatom, eine Methylgruppe, eine Carboxylgruppe oder eine konventionell geschützte Carboxylgruppe bedeutet, $R^3$ ein Wasserstoffatom oder eine Methylgruppe bedeutet, $R^{15}$ ein Wasserstoffatom oder eine konventionelle Carboxylschutzgruppe bedeutet, und X eine Gruppe

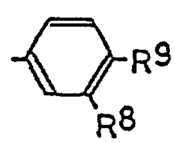

bedeutet, worin $R^8$ und $R^9$ gleich oder verschieden sind und Hydroxygruppen oder Alkoxygruppen bedeuten, umsetzt.

9. Verfahren zur Herstellung eines Cephalosporinderivats nach einem der Ansprüche 1 bis 5, dadurch gekennzeichnet, daß man eine Verbindung der Formel (V)

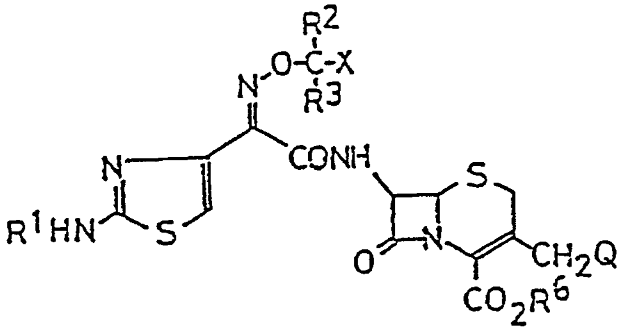

(V)

worin $R^1$ ein Wasserstoffatom oder eine konventionelle Aminoschutzgruppe bedeutet, $R^2$ ein Wasserstoffatom, eine Methylgruppe, eine Carboxylgruppe, oder eine konventionell geschützte Carboxylgruppe bedeutet, $R^3$ ein Wasserstoffatom oder eine Methylgruppe bedeutet, $R^6$ ein Wasserstoffatom oder eine konventionelle Carboxylschutzgruppe bedeutet, und Q ein Chloratom, ein Bromatom, ein Jodatom oder eine Acetoxygruppe bedeutet, und X eine Gruppe

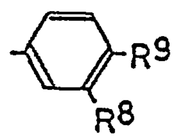

bedeutet, worin $R^8$ und $R^9$ gleich oder verschieden sind und Hydroxylgruppen oder Acetoxygruppen bedeuten, mit einer Verbindung der Formel (VI)

H–Y (VI)

worin H ein Wasserstoffatom bedeutet, und Y eine Gruppe

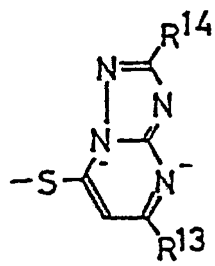

bedeutet, worin $R^{13}$ eine Methylgruppe bedeutet und $R^{14}$ eine Carboxylgruppe oder eine konventionell geschützte Carboxylgruppe bedeutet, umsetzt.

10. Verfahren zur Herstellung einer Cephalosporinverbindung nach einem der Ansprüche 1 bis 5, dadurch gekennzeichnet, daß man eine Verbindung der Formel (VII)

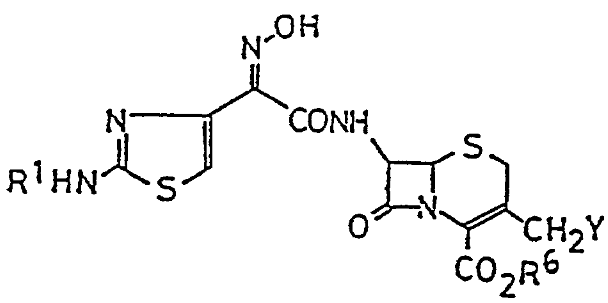

(VII)

worin $R^1$ ein Wasserstoffatom oder eine konventionelle Aminoschutzgruppe bedeutet, $R^6$ ein Wasserstoffatom oder eine konventionelle Carboxylschutzgruppe bedeutet, Y eine Gruppe

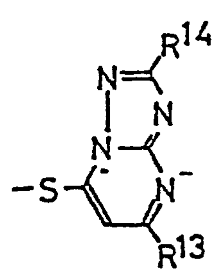

bedeutet, worin $R^{13}$ eine Methylgruppe bedeutet und $R^{14}$ eine Carboxylgruppe, eine konventionell geschützte Carboxylgruppe bedeutet oder ein Salz davon mit einer Verbindung der Formel (VIII)

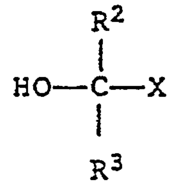

(VIII)

worin $R^2$ ein Wasserstoffatom, eine Methylgruppe, Carboxylgruppe oder eine konventionell geschützte Carboxylgruppe bedeutet, $R^3$ ein Wasserstoffatom oder eine Methylgruppe bedeutet und X eine Gruppe

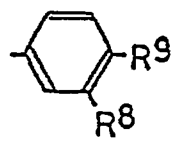

bedeutet, worin $R^8$ und $R^9$ gleich oder verschieden sind und Hydroxygruppen oder Acetoxygruppen bedeuten, umsetzt.

11. Verwendung eines Cephalosporinderivats nach einem der Ansprüche 1 bis 5 zur Herstellung eines antibakteriellen Mittels.

**Patentansprüche für den Vertragsstaat: AT**

1. Verwendung eines Cephalosporinderivats der allgemeinen Formel (I)

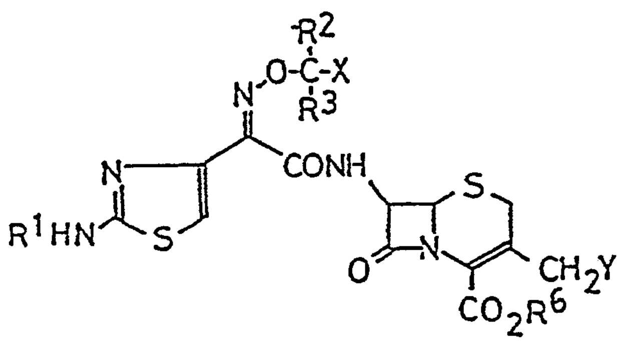

(I)

oder eines pharmazeutisch oder veterinär-medizinisch annehmbaren nicht-toxischen Salzes oder Solvats oder eines nicht-toxischen Salzes eines Solvats davon; worin $R^1$ ein Wasserstoff oder eine konventionelle Aminoschutzgruppe bedeutet, $R^2$ ein Wasserstoffatom, eine Methylgruppe, eine Carboxylgruppe oder eine konventionell geschützte Carboxylgruppe bedeutet, $R^3$ ein Wasserstoffatom oder eine Methylgruppe bedeutet, $R^6$ ein Wasserstoffatom oder eine konventionelle Carboxylschutzgruppe bedeutet, X eine Gruppe

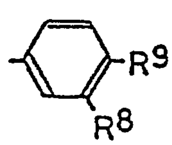

bedeutet, worin $R^8$ und $R^9$ gleich oder verschieden sind und Hydroxylgruppen oder Acetoxygruppen bedeuten und Y eine Gruppe

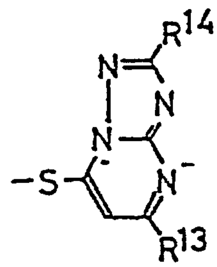

bedeutet, worin $R^{13}$ eine Methylgruppe bedeutet und $R^{14}$ eine Carboxylgruppe oder eine konventionell geschützte Carboxylgruppe bedeutet, zur Herstellung eines antibakteriellen Mittels.

2. Verwendung nach Anspruch 1, dadurch gekennzeichnet, daß $R^1$ und $R^6$ Wasserstoffatome darstellen.

3. Verwendung nach Anspruch 1 oder Anspruch 2, dadurch gekennzeichnet, daß $R^2$ eine Carboxylgruppe oder eine konventionell geschützte Carboxylgruppe darstellt.

4. Verwendung nach einem der vorhergehenden Ansprüche, dadurch gekennzeichnet, daß $R^2$ eine Carboxylgruppe bedeutet, $R^3$ ein Wasserstoffatom bedeutet, $R^8$ und $R^9$ Hydroxygruppen bedeuten, $R^{13}$ eine Methylgruppe bedeutet und $R^{14}$ eine Carboxylgruppe bedeutet.

5. Verwendung nach einem der vorhergehenden Ansprüche, dadurch gekennzeichnet, daß die absolute Konfiguration des Kohlenstoffatoms, an das $R^2$ und $R^3$ gebunden sind, die (S)-Konfiguration ist.

6. Verfahren zur Herstellung eines Cephalosporinderivats der allgemeinen Formel (I)

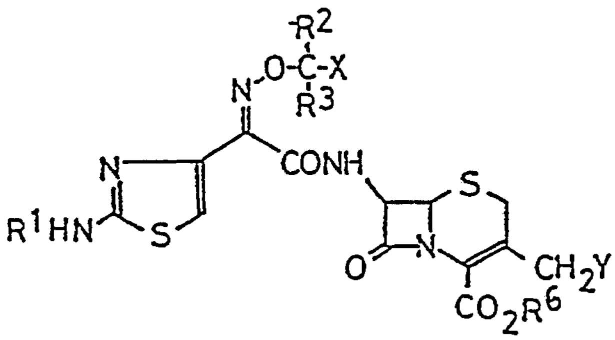

(I)

oder eines pharmazeutisch oder veterinär-medizinisch annehmbaren nicht-toxischen Salzes, oder Solvates oder eines nicht-toxischen Salzes eines Solvates davon; worin $R^1$, $R^2$, $R^3$, $R^6$, X und Y die in Anspruch 1 angegebene Bedeutung besitzen, dadurch gekennzeichnet, daß man eine Verbindung der Formel (III)

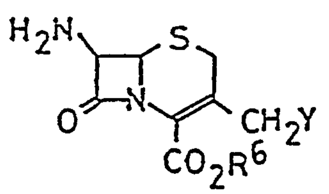

(III)

worin $R^6$ ein Wasserstoffatom oder eine konventionelle Carboxylschutzgruppe bedeutet und Y eine Gruppe

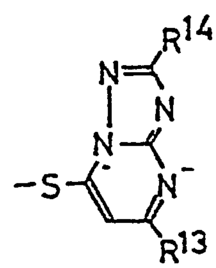

bedeutet, worin $R^{13}$ eine Methylgruppe bedeutet, und $R^{14}$ eine Carboxylgruppe oder eine konventionell geschützte Carboxylgruppe bedeutet, oder ein Salz davon mit einer Verbindung der Formel (IV)

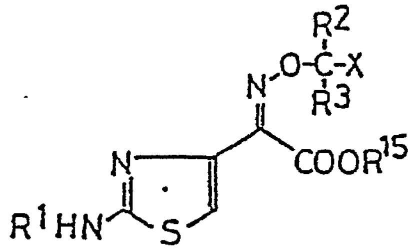

(IV)

worin $R^1$ ein Wasserstoffatom oder eine konventionelle Aminoschutzgruppe bedeutet, $R^2$ ein Wasserstoffatom, eine Methylgruppe, eine Carboxylgruppe oder eine konventionell geschützte Carboxylgruppe bedeutet, $R^3$ ein Wasserstoffatom oder eine Methylgruppe bedeutet, $R^{15}$ ein Wasserstoffatom oder eine konventionelle Carboxylschutzgruppe bedeutet, und X eine Gruppe

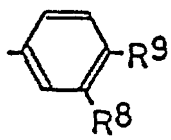

bedeutet, worin $R^8$ und $R^9$ gleich oder verschieden sind und Hydroxygruppen oder Acetoxygruppen bedeuten, umsetzt.

7. Verfahren zur Herstellung eines Cephalosporinderivats der allgemeinen Formel (I)

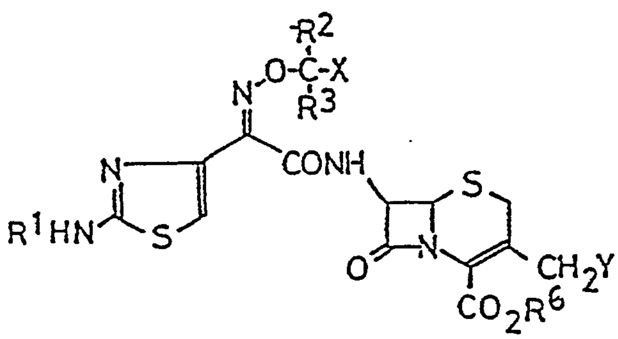

(I)

oder eines pharmazeutisch oder veterinär-medizinisch annehmbaren nicht-toxischen Salzes, oder Solvates oder eines nicht-toxischen Salzes eines Solvates davon; worin $R^1$, $R^2$, $R^3$, $R^6$, X und Y die in Anspruch 1 angegebene Bedeutung besitzen, dadurch gekennzeichnet, daß man eine Verbindung der Formel (V)

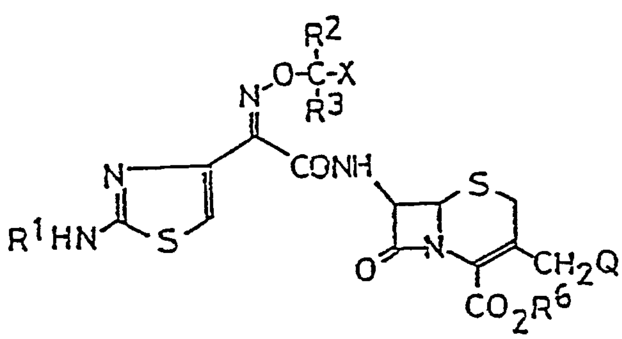

(V)

worin $R^1$ ein Wasserstoffatom oder eine konventionelle Aminoschutzgruppe bedeutet, $R^2$ ein Wasserstoffatom, eine Methylgruppe, eine Carboxylgruppe oder eine konventionell geschützte Carboxylgruppe bedeutet, $R^3$ ein Wasserstoffatom oder eine Methylgruppe bedeutet, $R^6$ ein Wasserstoffatom oder

eine konventionelle Carboxylschutzgruppe bedeutet, Q ein Chloratom, ein Bromatom, ein Jodatom oder eine Acetoxygruppe bedeutet, und X eine Gruppe

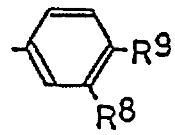

bedeutet, worin $R^8$ und $R^9$ gleich oder verschieden sind und Hydroxygruppen oder Acetoxygruppen bedeuten, mit einer Verbindung der Formel (VI):

H–Y (VI)

worin H ein Wasserstoffatom bedeutet und Y eine Gruppe:

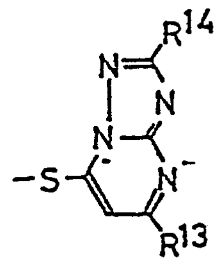

bedeutet, worin $R^{13}$ eine Methylgruppe bedeutet und $R^{14}$ eine Carboxylgruppe oder eine konventionell geschützte Carboxylgruppe bedeutet, umsetzt.

8. Verfahren zur Herstellung eines Cephalosporinderivats der allgemeinen Formel (I)

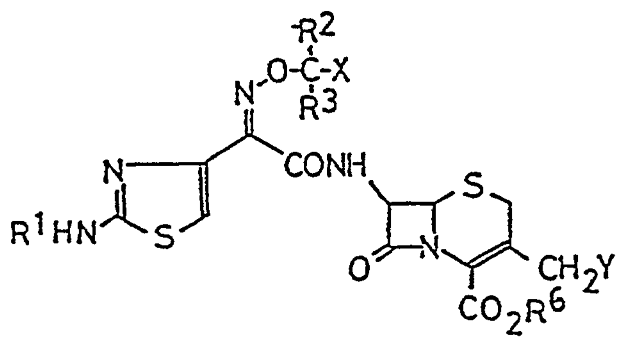

(I)

oder eines pharmazeutisch oder veterinär-medizinisch annehmbaren nicht-toxischen Salzes, oder Solvates, oder eines nicht-toxischen Salzes eines Solvates davon, worin $R^1$, $R^2$, $R^3$, $R^6$, X und Y die in Anspruch 1 angegebene Bedeutung besitzen, dadurch gekennzeichnet, daß man eine Verbindung der Formel (VII):

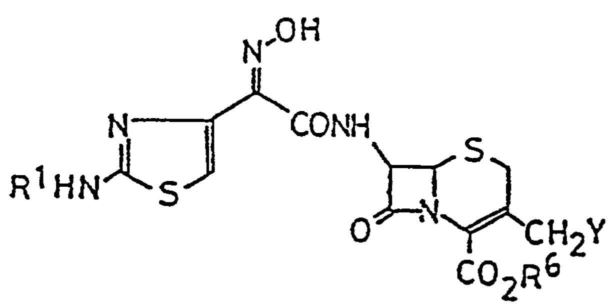

(VII)

worin $R^1$ ein Wasserstoffatom oder eine konventionelle Aminoschutzgruppe bedeutet, $R^6$ ein Wasserstoffatom oder eine konventionelle Carboxylschutzgruppe bedeutet, Y eine Gruppe

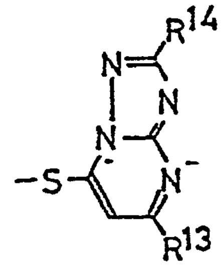

bedeutet, worin $R^{13}$ eine Methylgruppe bedeutet und $R^{14}$ eine Carboxylgruppe, eine konventionell geschützte Carboxylgruppe bedeutet, oder ein Salz davon mit einer Verbindung der Formel (VIII):

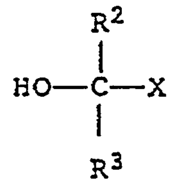

(VIII)

worin $R^2$ ein Wasserstoffatom, eine Methylgruppe, Carboxylgruppe oder eine konventionell geschützte Carboxylgruppe bedeutet, $R^3$ ein Wasserstoffatom oder eine Methylgruppe bedeutet, und X eine Gruppe

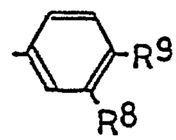

bedeutet, worin $R^8$ und $R^9$ gleich oder verschieden sind und Hydroxylgruppen oder Acetoxygruppen bedeuten, umsetzt.

9. Cephalosporinderivat der allgemeinen Formel (I):

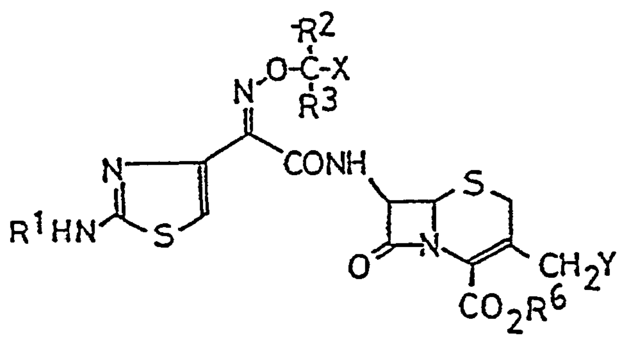

(I)

oder ein pharmazeutisch oder veterinär-medizinisch annehmbares nicht-toxisches Salz, oder Solvat, oder eines nicht-toxischen Salzes eines Solvates davon, worin $R^1$, $R^2$, $R^3$, $R^6$, X und Y die in Anspruch 1 angegebene Bedeutung besitzen.

10. Pharmazeutische Zusammensetzung umfassend eine pharmazeutisch oder veterinär-medizinisch annehmbare Trägersubstanz und mindestens ein Cephalosporinderivat nach Anspruch 9.

11. Pharmazeutische Zusammensetzung nach Anspruch 10, dadurch gekennzeichnet, daß sie eine pharmazeutisch oder veterinär-medizinisch wirksame Menge von mindestens einem Cephalosporinderivat enthält.

**Revendications pour les Etats contractants: BE, CH, LI, DE, FR, GB, IT, LU, NL, SE**

1. Un dérivé de céphalosporine représenté par la formule générale (I):

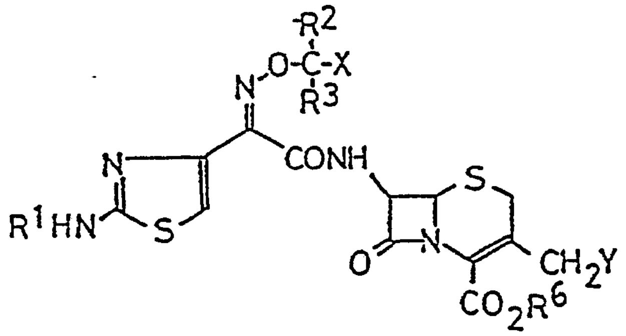

(I)

ou un sel non toxique convenant à l'usage pharmaceutique ou vétérinaire ou un solvate ou un sel non toxique d'un solvate de celui-ci; dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^6$ représente un atome d'hydrogène ou un groupe carboxyl-protecteur classique, X représente un groupe:

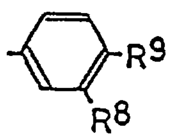

dans lequel $R^8$ et $R^9$ sont semblables ou différents et représentent des groupes hydroxy ou des groupes acétoxy, et Y représente un groupe:

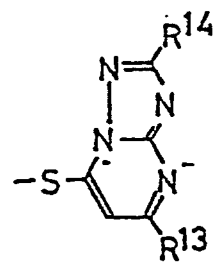

où $R^{13}$ représente un groupe méthyle et $R^{14}$ représente un groupe carboxyle ou un groupe carboxyle classiquement protégé.

2. Un dérivé de céphalosporine selon la revendication 1, où $R^1$ et $R^2$ représentent des atomes d'hydrogène.

3. Un dérivé de céphalosporine selon la revendication 1 ou la revendication 2, où $R^2$ représente un groupe carboxyle ou un groupe carboxyle classiquement protégé.

4. Un dérivé de céphalosporine selon l'une quelconque des revendications précédentes, où $R^2$ représente un groupe carboxyle, $R^3$ représente un atome d'hydrogène, $R^8$ et $R^9$ représentent des groupes hydroxy, $R^{13}$ représente un groupe méthyle et $R^{14}$ représente un groupe carboxyle.

5. Un dérivé de céphalosporine selon l'une quelconque des revendications précédentes, où la configuration absolue de l'atome de carbone auquel $R^2$ et $R^3$ sont raccordés est la configuration (S).

6. Une composition pharmaceutique qui comprend un véhicule convenant à l'usage pharmaceutique ou vétérinaire et au moins un dérivé de céphalosporine selon l'une quelconque des revendications 1 à 5.

7. Une composition pharmaceutique selon la revendication 6 comprenant une quantité efficace, du point de vue pharmaceutique ou vétérinaire, d'au moins un dérivé de céphalosporine.

8. Un procédé pour préparer un dérivé de céphalosporine selon l'une quelconque des revendications 1 à 5, comprenant la réaction d'un composé représenté par la formule (III):

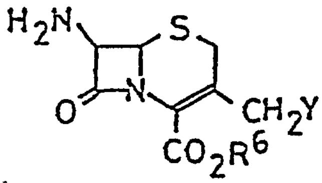 (III)

dans laquelle $R^6$ représente un atome d'hydrogène ou un groupe carboxyl-protecteur classique et Y représente un groupe:

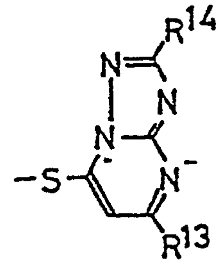

dans lequel $R^{13}$ représente un groupe méthyle et $R^{14}$ représente un groupe carboxyle ou un groupe carboxyle classiquement protégé ou un sel de celui-ci, avec un composé représenté par la formule (IV):

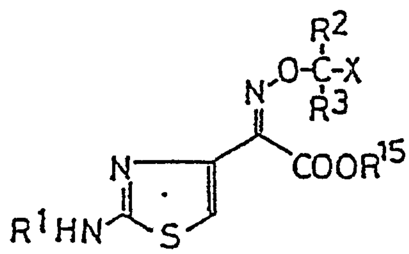 (IV)

dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^{15}$ représente un atome d'hydrogène ou un groupe carboxyl-protecteur classique et X représente un groupe:

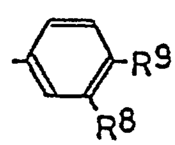

dans lequel $R^8$ et $R^9$ sont semblables ou différents et représentent des groupes hydroxy ou acétoxy.

9. Un procédé pour préparer un dérivé de céphalosporine selon l'une quelconque des revendications 1 à 5, comprenant la réaction d'un composé représenté par la formule (V):

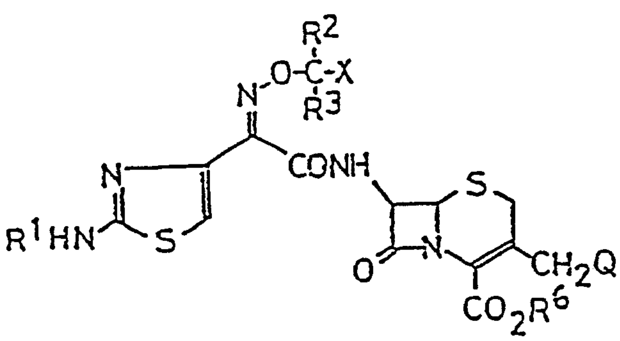 (V)

dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^6$ représente un atome d'hydrogène ou un groupe carboxyl-protecteur classique, Q représente un atome de chlore, un atome de brome, un atome d'iode ou un groupe acétoxy et X représente un groupe:

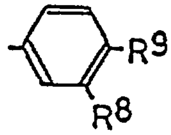

dans lequel $R^8$ et $R^9$ sont semblables ou différents et représentent des groupes hydroxy ou des groupes acétoxy, avec un composé représenté par la formule (VI):

H–Y (VI)

dans laquelle H représente un atome d'hydrogène et Y représente un groupe:

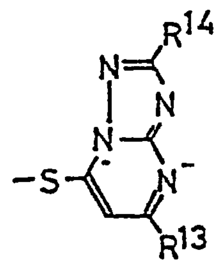

dans lequel $R^{13}$ représente un groupe méthyle et $R^{14}$ représente un groupe carboxyle ou un groupe carboxyle classiquement protégé.

10. Un procédé pour préparer un dérivé de céphalosporine selon l'une quelconque des revendications 1 à 5, comprenant la réaction d'un composé représenté par la formule (VII):

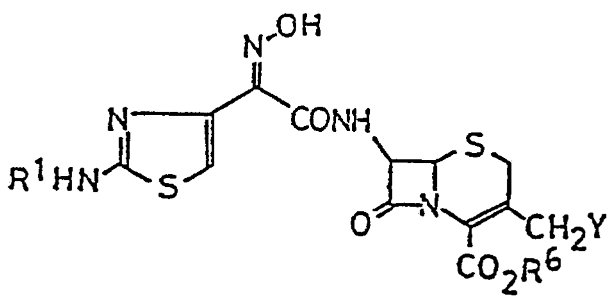

(VII)

dans laquelle $R^1$ représente un atome d'hydrogène ou un groupe amino-protecteur classique, $R^6$ représente un atome d'hydrogène ou un groupe carboxyl-protecteur classique et Y représente un groupe:

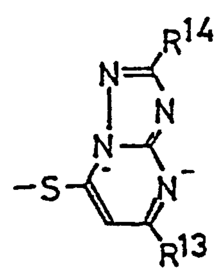

dans lequel $R^{13}$ représente un groupe méthyle et $R^{14}$ représente un groupe carboxyle, un groupe carboxyle classiquement protégé ou un sel de celui-ci, avec un composé représenté par la formule (VIII):

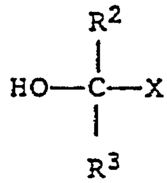

(VIII)

dans laquelle $R^2$ représente un atome d'hydrogène, un groupe méthyle, un groupe carboxyle ou un groupe carboxyle classiquement protégé, $R^3$ représente un atome d'hydrogène ou un groupe méthyle et X représente un groupe:

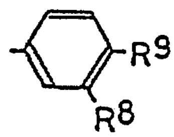

dans lequel $R^8$ et $R^9$ sont semblables ou différents et représentent des groupes hydroxy ou des groupes acétoxy.

11. L'utilisation d'un dérivé de céphalosporine selon l'une quelconque des revendications 1 à 5 pour la fabrication d'un agent antibactérien.

**Revendications pour l'Etat contractant: AT**

1. L'utilisation d'un dérivé de céphalosporine représenté par la formule générale (I):

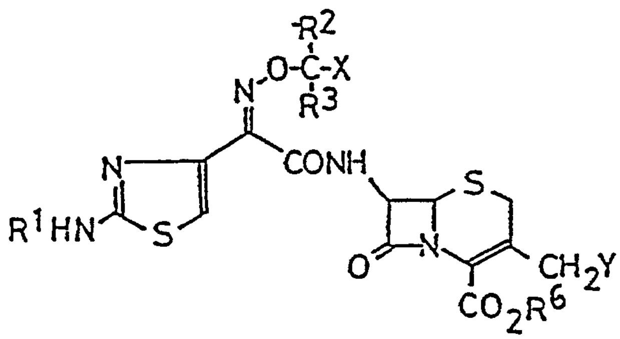

(I)

ou un sel non toxique convenant à l'usage pharmaceutique ou vétérinaire ou un solvate ou un sel non toxique d'un solvate de celui-ci; dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^6$ représente un atome d'hydrogène ou un groupe carboxyle-protecteur classique, X représente un groupe:

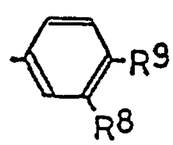

dans lequel $R^8$ et $R^9$ sont semblables ou différents et représentent des groupes hydroxy ou des groupes acétoxy, et Y représente un groupe:

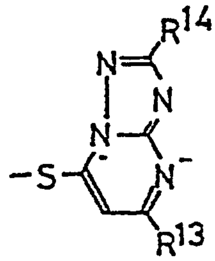

où $R^{13}$ représente un groupe méthyle et $R^{14}$ représente un groupe carboxyle ou un groupe carboxyle classiquement protégé, pour la fabrication d'un agent antibactérien.

2. L'utilisation selon la revendication 1, où $R^1$ et $R^6$ représentent des atomes d'hydrogène.

3. L'utilisation selon la revendication 1 ou la revendication 2, où $R^2$ représente un groupe carboxyle ou un groupe carboxyle classiquement protégé.

4. L'utilisation selon l'une quelconque des revendications précédentes, où $R^2$ représente un groupe carboxyle, $R^3$ représente un atome d'hydrogène, $R^8$ et $R^9$ représentent des groupes hydroxy, $R^{13}$ représente un groupe méthyle et $R^{14}$ représente un groupe carboxyle.

5. L'utilisation selon l'une quelconque des revendications précédentes, où la configuration absolue de l'atome de carbone auquel $R^2$ et $R^3$ sont raccordés est la configuration (S).

6. Un procédé pour préparer un dérivé de céphalosporine représenté par la formule générale (I):

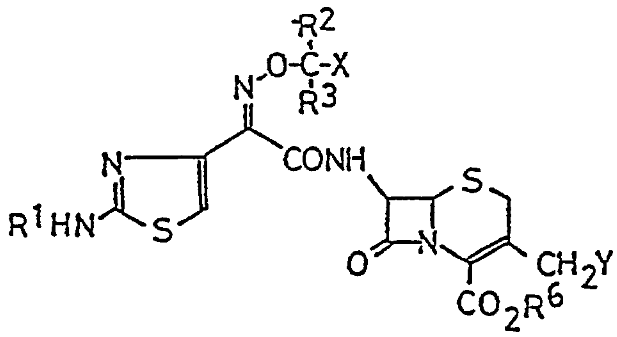

(I)

ou un sel non toxique convenant à l'usage pharmaceutique ou vétérinaire ou un solvate ou un sel non toxique d'un solvate de celui-ci; dans laquelle $R^1$, $R^2$, $R^3$, $R^6$, X et Y sont comme défini dans la revendication 1, comprenant la réaction d'un composé représenté par la formule (III):

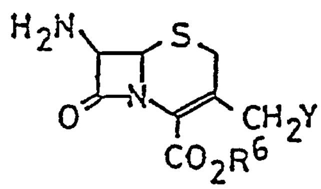

(III)

dans laquelle $R^6$ représente un atome d'hydrogène ou un groupe carboxyl-protecteur classique et Y représente un groupe:

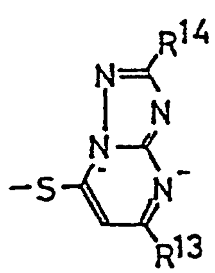

dans lequel $R^{13}$ représente un groupe méthyle et $R^{14}$ représente un groupe carboxyle ou un groupe carboxyle classiquement protégé ou un sel de celui-ci, avec un composé représenté par la formule (IV):

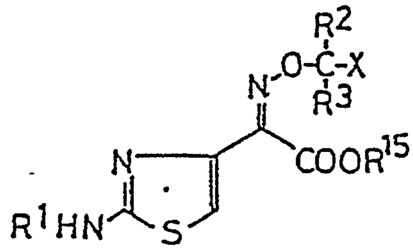

(IV)

dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^{15}$ représente un atome d'hydrogène ou un groupe carboxyl-protecteur classique et X représente un groupe:

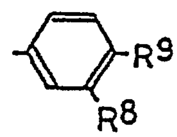

dans lequel $R^8$ et $R^9$ sont semblables ou différents et représentent des groupes hydroxy ou acétoxy.

7. Un procédé pour préparer un dérivé de céphalosporine représenté par la formule générale (I):

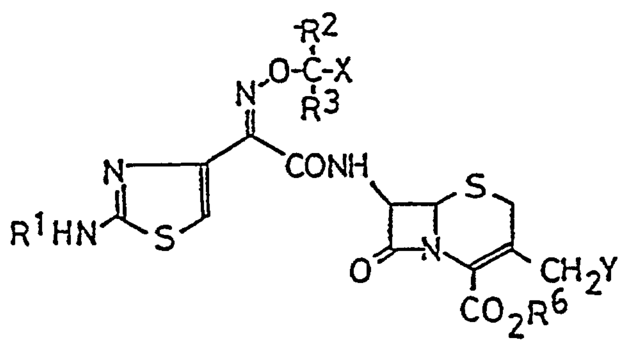

(I)

ou un sel non toxique convenant à l'usage pharmaceutique ou vétérinaire ou un solvate ou un sel non toxique d'un solvate de celui-ci; dans laquelle $R^1$, $R^2$, $R^3$, $R^6$, X et Y sont comme défini dans la revendication 1, comprenant la réaction d'un composé représente par la formule (V):

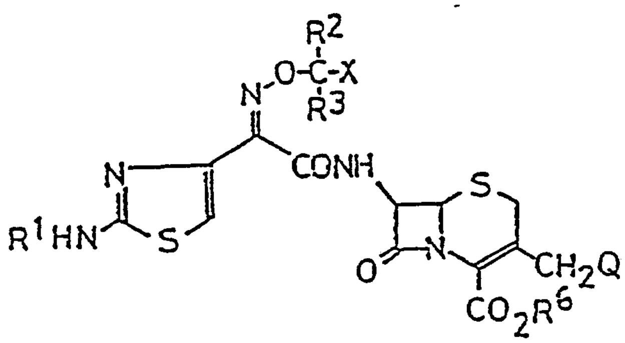

(V)

dans laquelle $R^1$ représente un atome d'hydrogène ou un groupe amino-protecteur classique, $R^2$ représente un atome d'hydrogène, un groupe méthyle, un groupe carboxyle ou un groupe carboxyle classiquement protégé, $R^3$ représente un atome d'hydrogène ou un groupe méthyle, $R^6$ représente un atome d'hydrogène ou un groupe carboxyl-protecteur classique, Q représente un atome de chlore, un atome de brome, un atome d'iode ou un groupe acétoxy et X représente un groupe:

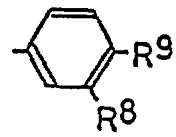

dans lequel $R^8$ et $R^9$ sont semblables ou différents et représentent des groupes hydroxy ou des groupes acétoxy, avec un composé représenté par la formule (VI):

H–Y (VI)

dans laquelle H représente un atome d'hydrogène et Y représente un groupe:

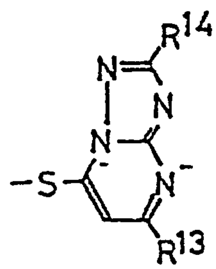

dans lequel $R^{13}$ représente un groupe méthyle et $R^{14}$ représente un groupe carboxyle ou un groupe carboxyle classiquement protégé.

8. Un procédé pour préparer un dérivé de céphalosporine représenté par la formule générale (I):

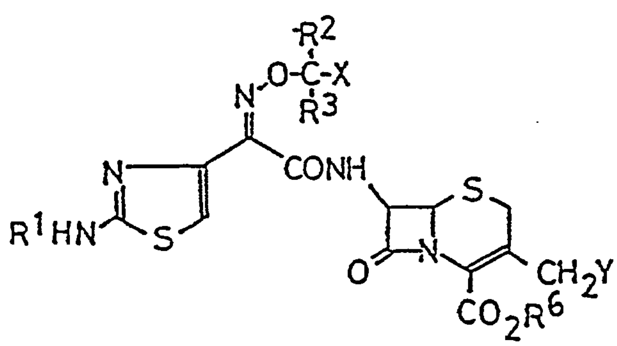

(I)

ou un sel non toxique convenant à l'usage pharmaceutique ou vétérinaire ou un solvate ou un sel non toxique d'un solvate de celui-ci; dans laquelle $R^1$, $R^2$, $R^3$, $R^6$, X et Y sont comme défini dans la revendication 1, comprenant la réaction d'un composé représenté par la formule (VII):

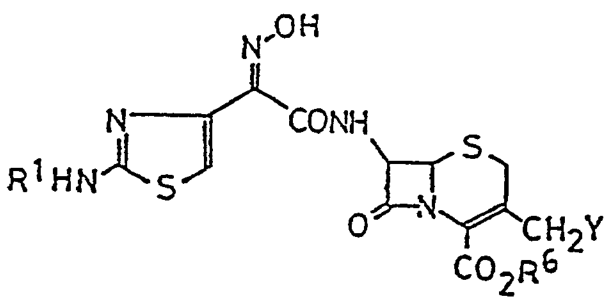

(VII)

dans laquelle $R^1$ représente un atome d'hydrogène ou un groupe amino-protecteur classique, $R^6$ représente un atome d'hydrogène ou un groupe carboxyl-protecteur classique et Y représente un groupe:

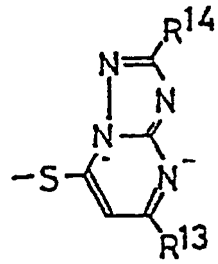

dans lequel $R^{13}$ représente un groupe méthyle et $R^{14}$ représente un groupe carboxyle, un groupe carboxyle classiquement protégé ou un sel de celui-ci, avec un composé représenté par la formule (VIII):

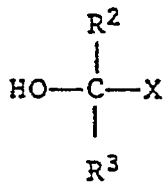

(VIII)

dans laquelle $R^2$ représente un atome d'hydrogène, un groupe méthyle, un groupe carboxyle ou un groupe carboxyle classiquement protégé, $R^3$ représente un atome d'hydrogène ou un groupe méthyle et X représente un groupe:

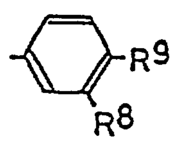

dans lequel $R^8$ et $R^9$ sont semblables ou différents et représentent des groupes hydroxy ou des groupes acétoxy.

9. Un dérivé de céphalosporine représenté par la formule générale (I):

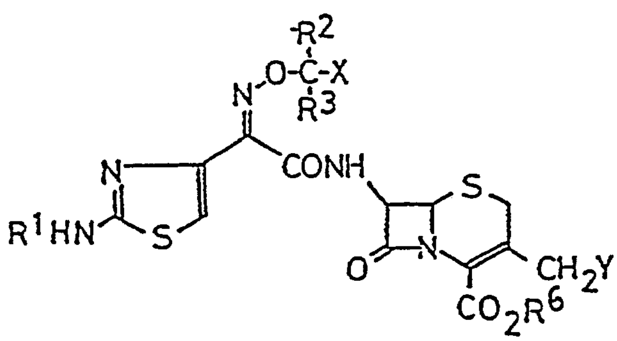

(I)

ou un sel non toxique convenant à l'usage pharmaceutique ou vétérinaire ou un solvate ou un sel non toxique d'un solvate de celui-ci; dans laquelle $R^1$, $R^2$, $R^3$, $R^6$, X et Y sont comme défini dans la revendication 1.

10. Une composition pharmaceutique qui comprend un véhicule convenant à l'usage pharmaceutique ou vétérinaire et au moins un dérivé de céphalosporine selon la revendication 9.

11. Une composition pharmaceutique selon la revendication 10 comprenant une quantité efficace pour l'usage pharmaceutique ou vétérinaire d'au moins un dérivé de céphalosporine.